# EUROPEAN PATENT APPLICATION

(11) **EP 4 803 537 A1**
(43) Date of publication of application: **09.09.2026**
(21) Application number: 24884984.6
(22) Date of filing: 01.11.2024
(51) Int. Cl.: C07K 16/00, A61K 39/395, C12N 15/13, A61P 37/02, A61P 35/00, C07K 16/46

(54) **FC REGION-BASED HETERODIMER MOLECULE AND USE THEREOF**

(30) Priority: 03.11.2023 WO PCT/CN2023/129691
(71) Applicant: PHRONTLINE BIOPHARMA (SUZHOU) CO., LTD, Jiangsu 215124 (CN); PHRONTLINE BIOPHARMA CO., LTD., Shanghai 200032 (CN)
(72) Inventor: HU, Siyi, Suzhou, Jiangsu 215124 (CN); CHEN, Zhaoyuan, Suzhou, Jiangsu 215124 (CN); HUANG, Mao, Shanghai 201203 (CN)
(74) Representative: Sagittarius IP
(86) International application number: PCT/CN2024/129416
(87) International publication number: WO 2025/092987

(57) **Abstract**

The present disclosure relates to heterodimeric Fc scaffolds based on the Fc region of an immunoglobulin, heteromultimeric proteins comprising said heterodimeric Fc scaffolds, in particular mono- or multi-specific binding proteins, and compositions comprising the same and uses thereof.

## Description

### Technical Field

The present disclosure relates to heterodimeric Fc scaffolds based on the Fc region of an immunoglobulin, heteromultimeric proteins comprising said heterodimeric Fc scaffolds, in particular mono- or multi-specific binding proteins, and compositions comprising the same and uses thereof.

### Background

Bispecific antibodies are a class of novel antibody drugs that have been rapidly developing in recent years. These antibodies simultaneously bind to two different antigens or to two different epitopes on the same antigen, thus holding promise for providing additional clinical benefits in the treatment of complex diseases.

Currently, there are many different structural formats of bispecific antibodies, including non-IgG-like formats based on the fusion of antibody fragments such as scFv, Fab, VH, and VL, and IgG-like formats based on an Fc domain. Compared to non-IgG-like formats, bispecific antibodies with formats similar to conventional IgG structures exhibit better physicochemical properties, stability, and pharmacokinetic characteristics, and therefore have broad application prospects.

It is known that the Fc region of antibodies mediates dimerization, which plays a crucial role in maintaining antibody function in vivo. Several Fc heterodimerization technologies have been reported to facilitate the production of bispecific antibodies (Moore GL, Methods 2019, 154: 38-50).

For example, Carter et al. developed the knobs-into-holes (KIH) model, which has been successfully applied to the preparation of bispecific antibodies (Patent US7951917B1). In the approach, a specific amino acid with a small side chain at the CH3 domain interface of a first heavy chain is mutated to an amino acid with a large side chain (e.g., T366Y), while a specific amino acid at the CH3 domain interface of a second heavy chain is mutated to an amino acid with a small side chain (e.g., Y407T). This creates spatial complementarity between the mutated residues at the interface, thereby promoting the formation of Fc heterodimers. In addition to the exemplary mutations described above, more widely used mutation sets in this approach include combining a knob mutation T366W with hole mutations T366S, L368A, and Y407V, by which an Fc heterodimer proportion of 80-90% can be achieved. However, the knobs-into-holes model still has insufficient capability to suppress the formation of homodimer impurities.

Electrostatic steering is also widely used to promote Fc heterodimer formation. This method involves mutating specific amino acids in the CH3 domain of one heavy chain to positively charged amino acids (Lys or Arg), and mutating specific amino acids in the CH3 domain of the other heavy chain to negatively charged amino acids (Asp or Glu). The electrostatic attraction between the mutated residues promotes Fc heterodimer formation. For example, Kannam et al. generated heterodimers at a high ratio by introducing mutations D399K and E356K in the CH3 region of a first heavy chain and mutations K409D and K392D in the CH3 region of a second heavy chain (Patent US8592562B2). Igawa et al. also promoted Fc heterodimer formation by introducing mutations E356K and D399K in the CH3 region of a first heavy chain and mutations K439E and K409D in the CH3 region of a second heavy chain (Patent US10011858B2).

Genmab developed the DuoBody technology. This technology involves introducing an F405L mutation in the CH3 domain of the heavy chain of one antibody and a K409R mutation in the CH3 domain of the heavy chain of another antibody. By expressing and purifying these two antibodies separately and then mixing them in the presence of a reducing agent (such as 2-MEA), controlled Fab-arm exchange between the two antibodies is achieved. This technology produces Fc heterodimers at a proportion of about 90-95% (Patent US9150663B2).

The strand-exchange (SEED) technology has also been used to facilitate Fc heterodimer formation (Muda et al., Proteins Eng. Des. Sel. 2011, 24: 447-454). This approach leverages sequence differences between IgA and IgG in the CH3 domain to generate Fc heterodimers by pairing complementary CH3 domains, thereby reducing homodimer formation.

Zymeworks reported a scaffold structure for enhancing the stability of Fc heterodimers (patent application US20120149876A1). For example, in one scaffold design (1a), mutations T366I, K392M, and T394W are introduced into the CH3 region of one heavy chain, while mutations F405A and Y407A are incorporated into the CH3 region of the other heavy chain, yielding a scaffold with a Tm of 74 °C. In another design (2a), mutations L351Y and Y407A are introduced into the CH3 region of one heavy chain, and T366V and K409F into the CH3 region of the other heavy chain, yielding a scaffold with a Tm of 75.5 °C. Using these scaffolds, the proportion of Fc heterodimers exceeds 90%.

Although various strategies have been proposed for the formation of Fc heterodimers, these strategies have certain drawbacks in their application. For example, most bispecific antibodies containing Fc domains suffer from homologous mispairing and reduced thermal stability, which affect the developability of the antibody drug.

For example, it has been found that most Fc heterodimers generated using existing technologies exhibit lower thermal stability than wild-type IgG1 monoclonal antibodies. The CH3-domain Tm of wild-type IgG1 monoclonal antibodies is generally above 80 °C (Ionescu RM, J Pharm Sci. 2008, 97: 1414-1426). However, CH3 amino-acid mutations introduced through Fc heterodimerization technology can substantially reduce the melting temperature (Tm) of the CH3 domain. For instance, the CH3 heterodimer produced using the knobs-into-holes model (T366W/T366S, L368A, Y407V) has a Tm of 69.4 °C (Atwell S, J. Mol. Biol. 1997, 270: 26-35). The CH3 heterodimer produced by an electrostatic steering strategy (D399K, E356K/K409D, K392D) has a Tm of 68.8 °C (Gunasekaran K, J. Biol. Chem. 2010, 285: 19637-19646). For structurally complex antibody drugs, a lower Tm can adversely affect manufacturing and storage. Although introducing non-naturally occurring disulfide bonds (e.g., Y349C and S354C) into the knobs-into-holes model may improve the formation efficiency and thermal stability of Fc heterodimers, for some products-such as antibody-drug conjugates (ADCs)-additional disulfide bonds may negatively affect product quality.

Furthermore, the proportion of Fc heterodimers produced by existing technologies is typically in the range of 80-95%, with other byproducts including Fc monomers, Fc homodimers, and high-molecular-weight aggregates. These impurities must be further removed by methods such as ion-exchange chromatography, and in particular, removing impurities arising from mispairing of homologous heavy chains remains a challenge for downstream purification. Moreover, higher impurity levels reduce product recovery after purification, thereby increasing production costs. the higher the impurity level, the greater the product loss during purification, leading to lower post-purification recovery and, consequently, higher manufacturing costs.

Given the broad application prospects of IgG-like bispecific antibodies, there remains an urgent need for Fc heterodimerization technologies that better address chain mispairing and thermal stability of bispecific antibodies, thereby enhancing their druggability.

### Summary of Invention

The inventors have discovered that, in a scaffold structure based on a dimeric Fc region, introducing specific CH3 domain interface mutations in addition to knobs-into-holes (KIH) mutations enables antibodies containing this Fc scaffold to exhibit improved thermal stability compared with corresponding antibodies containing only KIH mutations. At the same time, the advantages conferred by the KIH mutations (particularly, the advantage of promoting the pairing of the desired heterodimeric heavy chains) are retained, and biological effects comparable to natural antibodies are preserved, including antigen-specific binding and binding to FcRn and FcγR receptors. Based on these findings, the inventors provide a mutation combination for Fc heterodimerization that reduces chain mispairing and increases the stability of the resulting antibody product. Application of the mutation combination of the invention can effectively improve the developability and stability of bispecific antibodies.

In a first aspect, the present disclosure provides a CH3 heterodimer and a heterodimeric Fc scaffold comprising the mutation combination of the invention.

In a second aspect, the present disclosure provides the use of the heterodimeric Fc scaffold and CH3 heterodimer of the invention as components for constructing heteromultimeric proteins, in particular mono- or multi-specific binding proteins, and further provides such heteromultimeric proteins comprising said Fc scaffold or CH3 heterodimer.

In a third aspect, the present disclosure provides, in particular, a binding protein comprising a heterodimeric Fc scaffold of the invention.

In a fourth aspect, the present disclosure provides isolated polynucleotides encoding the heterodimeric Fc scaffold, CH3 heterodimer, heteromultimeric protein, or binding protein of the invention; vectors (particularly expression vectors) comprising the isolated polynucleotides of the invention; and host cells comprising the isolated polynucleotides or vectors (particularly expression vectors) of the invention. The present disclosure further provides uses and methods for producing the heterodimeric Fc scaffold, CH3 heterodimer, heteromultimeric protein, and binding proteins of the invention using the polynucleotides, vectors, or host cells of the invention.

In a fifth aspect, the present disclosure provides compositions comprising a heteromultimeric protein or binding protein of the invention, as well as conjugates comprising a therapeutic agent conjugated to a heteromultimeric protein or binding protein of the invention. In some embodiments, the composition is a pharmaceutical composition comprising a heteromultimeric protein of the invention (in particular, a multispecific binding protein of the invention) and a pharmaceutically acceptable carrier.

In a sixth aspect, the present disclosure further provides methods of using, and uses of, the heteromultimeric proteins, binding proteins, compositions, and conjugates of the invention. In some embodiments, the present disclosure provides the heteromultimeric proteins, binding proteins, compositions, or conjugates of the invention for use as a medicament. In some embodiments, the present disclosure provides the use of the heteromultimeric proteins, binding proteins, compositions, or conjugates of the invention for treating a disease in an individual in need thereof. Specifically, the disease is a cancer. The present disclosure further provides the use of the heteromultimeric proteins, binding proteins, compositions, or conjugates of the invention in the manufacture of a medicament for treating a disease in an individual in need thereof; and a method of treating a disease in an individual with the heteromultimeric proteins, binding proteins, compositions, or conjugates of the invention, wherein the method comprises administering to the individual a therapeutically effective amount of the heteromultimeric protein, binding protein, composition, or conjugate of the invention. In some embodiments, the heteromultimeric protein, binding protein, composition, or conjugate is formulated in a pharmaceutically acceptable form. In some embodiments, the disease is a cancer. In some embodiments, the individual is preferably a mammal, particularly a human.

### Brief Description of Figures

Figure 1 shows a schematic representation of the local crystal structure of the CH3-CH3 region of an Fc dimer.
Figure 1A shows hydrophobic interactions at the CH3-CH3 interface of wild-type IgG1 (PDB: 1HZH).
Figure 1B shows hydrophobic interactions at the CH3-CH3 interface of the knobs-into-holes model (PDB: 4NQS). The left side of the figure represents the knob chain, and the right side represents the hole chain.
Figure 1C shows a 3D structure of the CH3-CH3 region comprising the M2 mutant combination, based on computer simulation. The Hole L351Y mutation forms new hydrogen bonds with Knob T366W and E357 amino acids (indicated by dashed lines).
Figure 1D shows a 3D structure of the CH3-CH3 region comprising the M3 mutant combination, based on computer simulation. The Knob K409D mutation and the Hole D399R mutation form a new hydrogen-bond network with multiple amino acids at the CH3-CH3 interface (indicated by dashed lines).
Figure 2 shows the non-reducing SDS-PAGE electrophoresis images of the VHH-Fc/Fc mutant combinations.
In Figure 2A, the transfection ratio of VHH-Fc to Fc plasmids was 1:1. In Figure 2B, the transfection ratio of VHH-Fc to Fc plasmids was 0.6:1. For each indicated mutant combination, the heterodimers (AB) and other impurities (including homodimers and free monomers) in the Protein A-purified products exhibited distinct migration distances due to differences in molecular weight.
Figure 3 shows the Tm values of the VHH-Fc/Fc mutant combinations determined by the DSC method.
   The Tm values of the samples were analyzed using micro-differential scanning calorimetry (Microcal PEAQ-DSC). Each mutant combination showed one to three Tm values based on the changes in Cp values, with the highest Tm value reflecting the stability of the CH3 domain.
Figure 4 shows the non-reducing SDS-PAGE electrophoresis images of the VHH-Fc/Fab-Fc mutant combinations.
   For each indicated mutant combination, the heterodimers (ABC) and other impurities (homodimers, half-antibodies, free monomers) in the Protein A-purified products exhibited distinct migration distances due to differences in molecular weight.
Figure 5 shows the non-reducing SDS-PAGE electrophoresis images of the Fab-Fc mutants.
   For each indicated mutant, the transfection ratio of heavy chain to light chain plasmids was 1:1.5. For each indicated mutant, the half-antibody monomers, disulfide-linked homodimers, and other impurities (free light chains and free heavy chains) in the Protein A-purified products exhibited distinct migration distances due to differences in molecular weight.

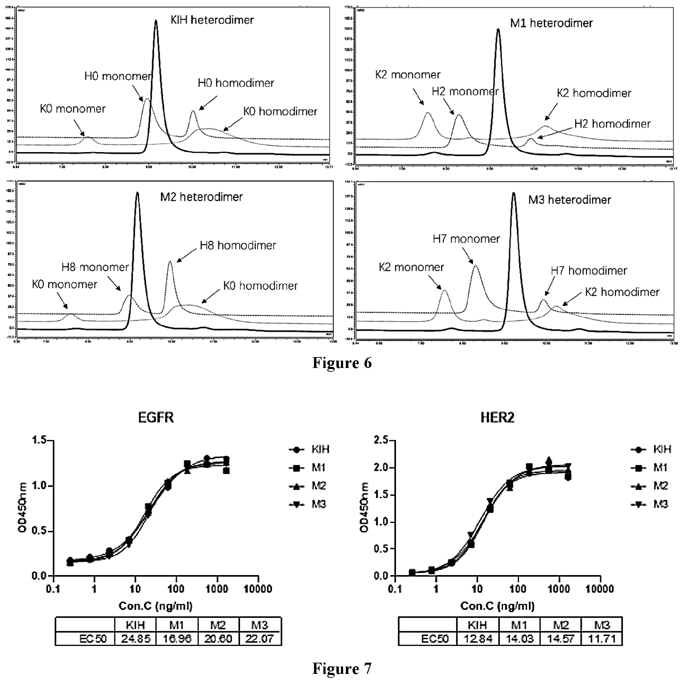

Figure 6 shows the *in vitro* assembly results of Fab-Fc mutant combinations detected by HIC-HPLC.
   Protein A affinity-purified half-antibodies exhibited two major peaks, corresponding to monomers and homodimers, respectively. Bispecific antibodies generated by *in vitro* assembly showed a single major peak corresponding to heterodimers, with minor peaks representing impurities of half-antibody monomers or homodimers.
Figure 7 shows the antigen-binding activity of the bispecific antibodies as determined by ELISA.

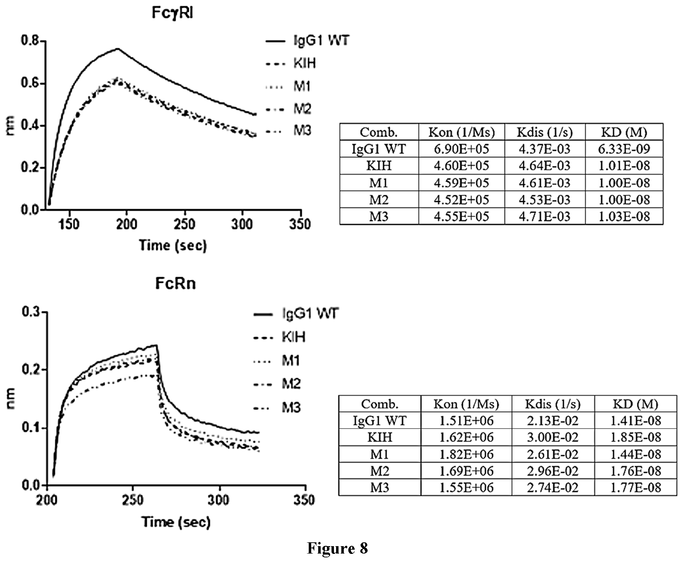

Figure 8 shows the binding affinity constants of the bispecific antibodies for FcRn and FcγRI, as determined by ForteBio.

### Detailed Description of the Invention

Unless otherwise defined, all technical and scientific terms used herein have the same meaning as commonly understood by one of ordinary skill in the art to which this invention pertains. All publications, patent applications, patents, and other references mentioned herein are incorporated by reference in their entirety. Furthermore, the materials, methods, and examples described herein are illustrative only and are not intended to be limiting. Other features, objects, and advantages of the invention will be apparent from the description and the drawings, and from the appended claims.

### I. Definitions

The term "about", when used in conjunction with a numerical value, refers to a range of numerical values having a lower limit that is 5% less than the specified numerical value and an upper limit that is 5% greater than the specified numerical value.

As used herein, the terms "comprising" and "including" mean including the recited elements, integers, or steps, but not excluding any other elements, integers, or steps. For example, when an Fc region is referred to as comprising a CH3 domain, this encompasses Fc regions that consist of, or consist essentially of, the CH3 domain, as well as Fc regions that further comprise other constant domain(s), such as CH2.

As used herein, the term "Fc scaffold" refers to a dimeric scaffold protein consisting, or consisting essentially of, immunoglobulin Fc regions that are paired and dimerized. This dimeric structure serves as a scaffold to which other functional molecules may be linked or conjugated at the N-terminus or the C-terminus, including, but not limited to, binding domains, biologically active polypeptides or fragments thereof, and toxins. In some aspects, the Fc scaffold is particularly useful as a building block for constructing multispecific (e.g., bispecific) antibodies or immunoadhesins.

As used herein, the term "heteromultimeric protein" refers to a molecule comprising at least a first polypeptide and a second polypeptide, wherein the second polypeptide differs from the first polypeptide in amino acid sequence by at least one amino acid residue. Preferably, the heteromultimeric protein is a monospecific or multispecific binding protein comprising at least one target-binding domain. In some aspects, the first and second polypeptides of the heteromultimeric protein comprise a multimerization domain (e.g., the first and second CH3 domains of a CH3 heterodimer of the invention; or the first and second Fc regions of a heterodimeric Fc scaffold of the invention), which facilitates the interaction of the first and second polypeptides at an interface to form a "heterodimer". In some aspects, the heteromultimeric protein may comprise a heterodimer formed by the first and second polypeptides, or may further comprise additional polypeptide(s) other than the first and second polypeptides, thereby forming a higher-order tertiary structure. Examples of heteromultimeric proteins include, but are not limited to, monospecific or multispecific binding proteins such as bispecific antibodies, bispecific immunoadhesins, and antibody/immunoadhesin chimeras.

As used herein, the terms "binding protein" and "binding molecule" are used interchangeably and in their broadest sense to refer to a protein molecule that specifically binds to at least one target. Examples of binding proteins include antibodies and immunoadhesins.

As used herein, the term "Fc scaffold-based binding protein" refers to a binding protein that comprises, consists of, or consists essentially of, an Fc scaffold and at least one target-binding domain linked thereto. In some embodiments, the binding protein comprises one (and only one) Fc scaffold. In other embodiments, the binding protein may comprise more than one Fc scaffold.

As used herein, the term "monospecific" refers to a binding molecule that specifically binds to one and only one particular target (e.g., an epitope). The term "multispecific" refers to a binding molecule that specifically binds to at least two distinct targets (e.g., at least two distinct epitopes). Typically, a multispecific binding molecule comprises at least two target-binding domains, each of which is specific for a distinct target. In some embodiments, the distinct targets may be polypeptides, proteins, or portions thereof that are present on the same cell or on different cells, or on the same antigen or on different antigens, such as receptors, ligands, or antigenic epitopes. In some embodiments, the multispecific binding molecule is bispecific, and is capable of simultaneously binding two distinct targets, such as two distinct epitopes; for example, two different epitopes expressed on two different cells, two different epitopes on two different antigens, or two different epitopes on the same antigen.

As used herein, the term "valency" refers to the number of target-binding domains present in a binding molecule. Accordingly, the term "monovalent," in relation to a binding molecule, means that there is one (and no more than one) target-specific binding domain in the binding molecule. Correspondingly, the term "multivalent" means that there are multiple target-specific binding domains in the binding molecule. For example, a "tetravalent" binding molecule refers to a binding molecule comprising a total of four target-specific binding domains, regardless of whether the targets recognized by the binding domains are the same.

As used herein, the term "target-binding domain" refers to the portion of a polypeptide or protein that mediates interaction with a target. Exemplary target-binding domains include an antigen-binding domain of an antibody, a ligand, a receptor-binding domain of a ligand, and a ligand-binding domain of a receptor. In some aspects of the present disclosure, a preferred target-binding domain is an antibody antigen-binding domain. An antibody antigen-binding domain generally comprises amino acid residues from the complementarity-determining regions (CDRs) of an antibody. Naturally occurring immunoglobulin molecules typically have two antigen-binding domains, while Fab molecules typically have a single antigen-binding domain.

As used herein, the term "antigen-binding domain" refers to an antibody or a fragment thereof that specifically binds to an antigenic epitope. In some specific embodiments, the antigen-binding domain comprises the heavy chain variable region (VH) and the light chain variable region (VL) of an antibody or, when the antibody is a heavy-chain antibody, a single heavy chain variable region (VHH). In some embodiments, the antigen-binding domain may further comprise an antibody constant region. The heavy chain constant region that may be used includes any of the following five classes: α, δ, ε, γ, and µ. The light chain constant region that may be used includes either of the following two types: κ and λ. Thus, in some aspects, the antigen-binding domain may be selected from, but is not limited to, an antibody fragment comprising the heavy chain variable region (VH) and the light chain variable region (VL) of an antibody, such as an Fv, an scFv, an Fab, an scFab, and an CrossFab, and an antibody fragment comprising the heavy chain variable region of a heavy-chain antibody, such as VHH.

As used herein, the terms "antigenic determinant" and "epitope" are used interchangeably and refer to a site on a macromolecule, such as a polypeptide or protein, that interacts with and forms a complex with an antigen-binding domain. In some aspects, an epitope may be, for example, a linear epitope composed of a continuous segment of amino acids, or a conformational epitope composed of non-contiguous amino acid residues located in different regions. According to the present disclosure, useful antigenic determinants may be found, for example, on the surface of tumor cells, virus-infected cells, other diseased cells, or immune cells, or on free macromolecules in body fluids and/or in the extracellular matrix (ECM).

As used herein, the term "antigen" refers to a macromolecule that is capable of eliciting an immune response in a mammal, thereby generating specific antibodies against the macromolecule. In some aspects, antigens useful in the present disclosure are polypeptides or proteins, including, but not limited to, naturally occurring vertebrate-derived proteins and variants, fragments, or derivatives thereof. Such vertebrates include mammals, for example, primates (e.g., humans).

As used herein, the term "immunoglobulin" refers to proteins that have the structure of naturally occurring antibodies. For example, IgG immunoglobulins are about 150,000 Dalton heterotetrameric glycoproteins composed of two light chains and two heavy chains linked by disulfide bonds. From the N-terminus to the C-terminus, each immunoglobulin heavy chain has one heavy chain variable region (VH), also called the heavy chain variable domain, followed by three heavy chain constant domains (CH1, CH2, and CH3), also called the heavy chain constant region. Similarly, from the N-terminus to the C-terminus, each immunoglobulin light chain has one light chain variable region (VL), also called the light chain variable domain, followed by one light chain constant domain (CL), also called the light chain constant region. In an IgG antibody molecule, the VH-CH1 of a heavy chain typically pairs with the VL-CL of a light chain to form an Fab fragment that specifically binds to an antigen. Therefore, an IgG immunoglobulin consists essentially of two Fab moieties linked to two dimerized Fc regions via an immunoglobulin hinge region. Based on the type of constant regions, immunoglobulin heavy chains are classified into five classes: α (IgA), δ (IgD), ε (IgE), γ (IgG) and µ (IgM), some of which are further divided into subclasses (e.g., γ1 (IgG1), γ2 (IgG2), γ3 (IgG3), γ4 (IgG4), α1 (IgA1) and α2 (IgA2)). Immunoglobulin light chains are classified into two types (κ and λ) based on the amino acid sequence of their constant domain.

As used herein, the term "antibody" refers to a polypeptide comprising one or more domains that bind to an epitope on an antigen of interest, wherein the binding domain has a variable region sequence derived from an immunoglobulin or has sequence identity thereto. This term encompasses various antibody structures, including, but not limited to, single-chain antibodies, mono- and multi-specific antibodies, chimeric antibodies, humanized antibodies, human sequence antibodies, full-length antibodies, and antibody fragments, as well as antigen-binding proteins assembled from any of the foregoing as components, provided that they exhibit the desired antigen-binding activity. Typically, an antigen-binding domain of an antibody is formed by three complementarity-determining regions (HCDRs 1-3) in the heavy chain variable region (VH) and three complementarity-determining regions (LCDRs 1-3) in the light chain variable region (VL) at the surface of the VH-VL dimer. These six CDRs confer specific binding of the antibody to the antigen. However, in the case of heavy-chain antibodies, such as those from camelids, the antibodies can confer specific binding to an antigen through three complementarity-determining regions (CDRs 1-3) in a single VH domain (also referred to herein as a VHH domain). The VHH domain, like the heavy and light chain variable regions of conventional IgG antibodies, contains four conserved framework regions (FRs) and three complementarity-determining regions (CDRs), arranged in the order FR1-CDR1-FR2-CDR2-FR3-CDR3-FR4.

As used herein, the terms "complementarity-determining region," "CDR region," "CDR," or "hypervariable region" refer to a region that is highly variable in sequence and forms a structurally defined loop ("hypervariable loop") and/or contains antigen-contact residues ("antigen-contact sites") within an antibody variable region. The CDRs are primarily responsible for binding to an antigenic epitope. In the VHH domains and VH/VL domains of the antibodies of the present disclosure, the CDRs are numbered sequentially from the N-terminus and are conventionally designated CDR1, CDR2, and CDR3. The CDR sequences within a defined VHH domain or a defined VH/VL domain may be determined using methods well known in the art. Those skilled in the art can readily determine the boundaries of CDR sequences for any given antibody variable region amino acid sequence at http://www.abysis.org/abysis/, including those defined by the Kabat, AbM, Chothia, Contact, and IMGT schemes, and combinations thereof. Unless otherwise indicated, the term "CDR" or "CDR sequence" used in the present disclosure encompass a CDR sequence determined by any of the foregoing methods and combinations thereof, and preferably a CDR sequence defined according to the Kabat scheme.

As used herein, the term "antibody fragment" refers to a portion of an antibody that is less than a full-length antibody, and that comprises an antigen-binding domain. Examples of antibody fragments include, but are not limited to, Fv, Fab, Fab', Fab'-SH, F(ab')₂, diabody, linear antibody, single-chain antibody (e.g., scFv, scFab), and single-domain antibody (sdAb). The amino acid sequence of the VH or VL within an antibody fragment may be modified by substitution, deletion, addition, and/or insertion, so long as antigen-binding activity is retained. Furthermore, a variable region of an antibody fragment may be chimerized or humanized.

As used herein, the term "Fv domain" refers to the smallest antibody fragment that contains a complete antigen recognition and binding site. "Fv" is a dimer (VH-VL dimer) formed by tight non-covalent association of a VH domain and a VL domain.

As used herein, the term "scFv domain" refers to a single-chain polypeptide in which two variable regions (typically a VH and a VL) are linked via a linker to form an antigen-binding domain required for antigen binding.

As used herein, the term "Fab domain" refers to an antigen-binding domain that, analogous to that in a conventional four-chain IgG antibody, is formed by pairing a heavy-chain variable region VH and heavy-chain constant region CH1 (VH-CH1) with a cognate light-chain variable region VL and light-chain constant region CL (VL-CL).

As used herein, the term "crossFab" or "crossFab domain" refers to a Fab domain in which CH1 and CL are exchanged, i.e., an antigen-binding domain formed by the pairing of VH-CL and VL-CH1.

As used herein, the term "scFab" refers to a Fab domain that is linked into a single polypeptide chain via an artificial linker.

As used herein, the term "VHH" or "VHH domain" refers to a heavy-chain variable domain derived from a heavy-chain antibody that lacks a light chain. Accordingly, a VHH differs from a conventional VH of a four-chain immunoglobulin in that it does not require pairing with a light-chain variable domain to form an antigen-binding domain. Such VHH molecules may be derived from antibodies produced by camelid species (e.g., camels, alpacas, dromedaries, llamas, and guanacos). Species other than camelids may also produce naturally occurring heavy-chain antibodies lacking a light chain, and such VHHs are also within the scope of the present disclosure. In some cases, for therapeutic applications of VHHs, it may be desirable to reduce immunogenicity. Accordingly, the VHH domain for use in the binding proteins of the invention preferably comprises a humanized sequence.

As used herein, the term "immunoglobulin constant domain" refers to a constant domain originating from, derived from, or obtained from a heavy chain (e.g., a human IgG1 heavy chain) or a light chain of an immunoglobulin, including heavy-chain constant domains CH1, CH2, CH3, and optionally CH4, and light-chain constant domain CL. The term encompasses both native-sequence and variant-sequence constant domains.

As used herein, the term "class" or "subclass" of an immunoglobulin constant domain refers to the class or subclass determined on the basis of its amino acid sequence. Heavy-chain constant domains can be classified into five distinct classes based on their sequences: IgA, IgD, IgE, IgG, and IgM, and may be further divided into subclasses (isotypes), such as IgG1, IgG2, IgG3, IgG4, IgA1, and IgA2. Accordingly, as used herein, an IgG1 Fc region or CH3 domain refers to an Fc region or CH3 domain that, based on its amino acid sequence, is classified as belonging to the IgG class, and further classified as belonging to the IgG1 subclass. Similarly, light-chain constant domains can be classified into κ and λ light-chain CL domains based on their sequences. Those skilled in the art can readily determine the class or subclass of a given constant domain by comparing its amino acid sequence with the sequences of corresponding constant domains of native immunoglobulins of different classes or subclasses. In the present disclosure, where a heterodimeric Fc scaffold or heteromultimeric protein molecule of the invention comprises multiple immunoglobulin heavy-chain constant domains, it will be understood that these domains may be selected independently of one another according to the intended function or use of the molecule. By way of example, for a heteromultimer of the invention comprising a CH1 domain together with CH2 and CH3 domains, all three constant domains may be of the IgG1 subclass, e.g., the human IgG1 subclass; or alternatively, only the CH3 domain, or the CH2 and CH3 domains, may be of the IgG1 subclass, e.g., the human IgG1 subclass.

As used herein, the term "IgG format" refers to an antibody that has the same architecture as that of an IgG immunoglobulin, consisting essentially of two Fab domains linked to the N-termini of a dimerized Fc domain via an immunoglobulin hinge region (or, where appropriate, via a flexible linker peptide). Thus, in a typical embodiment, an antibody in an IgG format consists of two heavy chains and two light chains, wherein each heavy chain comprises, from N-terminus to C-terminus, VH, CH1, CH2, and CH3 domains; and each light chain comprises, from N-terminus to C-terminus, VL and CL domains.

As used herein, the term "IgG-like format" refers to an antibody that retains the Y-shaped structural feature of an IgG immunoglobulin, but lacks one Fab domain and/or has at least one Fab domain replaced by a binding domain having a different configuration (e.g., an scFv, a VHH, a ligand, or a ligand-binding domain of a receptor). IgG-like format antibodies may be described by specifying the binding domain(s) linked to the dimerized Fc scaffold. For example, a VHH-Fc/Fc antibody is a monovalent IgG-like format antibody composed of a VHH-Fc polypeptide and an Fc polypeptide; a VHH-Fc/Fab-Fc antibody is a bivalent IgG-like format antibody composed of a VHH-Fc polypeptide and a Fab-Fc polypeptide.

As used herein, the term "immunoadhesin" refers to an antibody-like protein molecule formed by fusing a non-immunoglobulin binding domain having a desired binding specificity (e.g., a binding domain derived from a cell-surface receptor or ligand, or a ligand itself) to an immunoglobulin constant domain (e.g., an Fc region or CH3 domain). The immunoglobulin constant domain may be derived from any immunoglobulin, such as IgG, particularly IgG1, IgG2, IgG3, or IgG4.

As used herein, the term "Fc domain" or "Fc region" refers to a portion of the constant region of an immunoglobulin heavy chain that comprises an CH3 domain, or a fragment thereof. In some instances, the immunoglobulin portion may further comprise one or more additional immunoglobulin constant domains, or fragments thereof, including a hinge region, CH1, or CH2 domain. As used herein, amino acid residues in Fc regions and constant domains are numbered according to the EU numbering system (also referred to as the EU index) described in Kabat et al., Sequences of Proteins of Immunological Interest, 5th Edition, Public Health Service, National Institutes of Health, Bethesda, MD (1991). For a human IgG1 Fc region, the IMGT Scientific Chart may also be consulted for EU numbering of amino acid residues (https://www.imgt.org/IMGTScientificChart /Numbering/Hu_IGHGnber.html), which is incorporated herein by reference. Under the EU numbering system, in the heavy chain of a human IgG1 immunoglobulin, amino acid residues 118-215 correspond to the CH1 domain, residues 216-230 correspond to the hinge region, residues 231-340 correspond to the CH2 domain, and residues 341-447 correspond to the CH3 domain. In some instances, the C-terminal lysine residue (Lys447) of CH3 domain may be absent. Those skilled in the art can readily identify the constant domains present in a heterodimeric Fc region and in a binding protein of the present invention, as well as their classes/subclasses and species of origin, by sequence alignment to published constant-region sequences of native immunoglobulins. In some embodiments, the first and second Fc regions of a heterodimeric Fc scaffold of the invention comprise a CH3 domain, or consist of a CH3 domain. In some other embodiments, the Fc regions further comprise a CH2 domain. In some other embodiments, the Fc regions further comprise a hinge region or a portion thereof, for example, a lower hinge region comprising the sequence "CPPCP". As used herein, those skilled in the art will understand that a CH3 heterodimer according to the present invention is a particular embodiment of the heterodimeric Fc scaffold of the invention, in which the first and second Fc regions consist of, or consist essentially of, CH3 domains. Accordingly, unless expressly stated otherwise, descriptions applicable to the heterodimeric Fc scaffold apply equally to the CH3 heterodimer.

As used herein, the terms "Fc domain" and "Fc region" encompass both native-sequence and variant-sequence Fc regions. The term "native-sequence Fc region", as used herein, encompasses the Fc region sequences of various naturally occurring immunoglobulins, such as the Fc region sequences of various Ig subclasses and allotypes thereof (Gestur Vidarsson et al., IgG subclasses and allotypes: from structure to effector functions, 20 October 2014, doi: 10.3389/fimmu.2014.00520.). As used herein, the term "variant-sequence Fc region" refers to an Fc polypeptide comprising a modification relative to a native-sequence Fc region. The modification may be the addition, deletion, or substitution of amino acid residue(s). Substitution includes substitution with naturally occurring and non-naturally occurring amino acids. Such modification may be intended to alter the physicochemical properties of an Fc region, such as thermal stability and heterodimerization propensity, and/or to alter binding of an Fc region to its receptor(s) and/or its effector function(s).

As used herein, an "Fc scaffold" may also be referred to as an "Fc scaffold dimer" or a "dimeric Fc scaffold." The term "homodimeric" Fc scaffold, as used herein, refers to one in which the first and second Fc regions constituting the Fc scaffold are identical in sequence. Correspondingly, a "heterodimeric" Fc scaffold refers to one in which the first and second Fc regions constituting the scaffold differ in sequence by at least one amino acid residue.

As used herein, the term "CH3 dimer" refers to a pair of CH3 domains that are paired and dimerized. The term "CH3 homodimer" refers to a dimer in which the first and second CH3 domains are identical in sequence. Correspondingly, a "CH3 heterodimer" refers to a dimer in which the first and second CH3 domains differ in sequence by at least one amino acid residue.

As used herein, where an Fc scaffold-based heteromultimeric protein (e.g., a mono- or multispecific binding protein) comprises only one Fc scaffold, such multimeric protein may be regarded as a dimer composed of two protein units (i.e., monomers) with reference to the two Fc regions of the scaffold. One Fc member of the Fc scaffold, together with the polypeptide(s) linked thereto (e.g., target-binding domain(s), which may be zero, one, or more), constitutes one protein unit (i.e., monomer) of the dimer; and the other Fc member of the Fc scaffold, together with the polypeptide(s) linked thereto (e.g., target-binding domain(s), which may be zero, one, or more), constitutes the other protein unit (i.e., monomer) of the dimer. In this regard, it will be understood that each monomer constituting the dimer may comprise one or more polypeptide chains. When the two monomers are identical in sequence and structure (i.e., are copies of one another), the dimer is referred to herein as a homodimer. For example, a native IgG immunoglobulin is a typical example of such a homodimer. When the two monomers differ, e.g., in the Fc sequence and/or in the sequence of a binding domain linked thereto, the dimer is referred to herein as a heterodimer. For example, bispecific IgG antibodies or IgG-like antibodies are typical examples of such heterodimers. Mono- or multispecific antibodies based on heterodimeric Fc scaffolds represent another typical example of such heterodimers.

As used herein, the term "purity," when used in relation to a heteromultimeric protein of the invention (e.g., a binding protein of the invention), refers to the percentage of the heteromultimeric protein relative to the total protein in a purified preparation obtained after expression and purification of the heteromultimeric protein from host cells. The purity of the desired heteromultimeric protein can be determined by SEC-HPLC through measuring the proportions of the heteromultimeric protein and chain-mismatched products in the purified preparation. Preferably, the purity is greater than 90%.

As used herein, the term "thermal stability", when used in relation to a heteromultimeric protein of the invention (e.g., a binding protein of the invention), means that the protein exhibits a CH3 Tm value greater than 70°C as determined by DSC; or, where the protein is an antibody in an IgG format, that it exhibits a CH3 Tm value similar to that of a native human IgG1 immunoglobulin (e.g., ±2°C, preferably ±1°C). For heteromultimeric proteins of the invention based on heterodimeric Fc scaffolds, the Tm value is preferably determined under conditions that do not introduce non-naturally occurring disulfide bonds into the heterodimeric Fc scaffold.

As used herein, a single mutation is described by reference to the position of the amino acid residue at which the mutation occurs and the amino acid residues before and after the mutation, and is designated as [original amino acid residue] [residue position] [mutated amino acid residue]. For example, substitution of threonine with tryptophan at position 366 of an Fc region is designated as T366W; substitution of tyrosine with valine at position 407 of an Fc region is designated as Y407V. When referring to a combination of multiple mutations, mutations present on the same polypeptide chain are linked by a hyphen (-); for example, the combination of L351Y and D399R mutations on the hole chain may be represented as "L351Y-D399R". For a combination of mutations located on different polypeptide chains, the symbol "/" is used to separate them; for example, L351Y and D399R mutations on the hole chain in combination with the K409D mutation on the knob chain may be represented as "Hole L351Y-D399R/Knob K409D".

As used herein, the terms "flexible linking peptide" and "peptide linker" are used interchangeably and refer to a short amino acid sequence composed of amino acids such as glycine (G), serine (S), and/or threonine (T) residues, used alone or in combination, or to an immunoglobulin hinge region or a modified form thereof.

As used herein, the term "conjugate" refers to a modified form or derivative of a binding protein obtained by covalently linking or conjugating an additional molecule(s) (e.g., a therapeutic or diagnostic molecule(s)) to a desired protein (e.g., a heteromultimeric protein or binding protein of the invention). Examples of such additional molecules include, but are not limited to, proteins, polypeptides or peptides; labels; drugs; and cytotoxic agents, such as: radioisotopes; chemotherapeutic agents; growth inhibitors; enzymes and fragments thereof; fluorescent reporter proteins; antibiotics; toxins (e.g., small molecule toxins or enzymatically active toxins of bacterial, fungal, plant, or animal origin, including fragments and/or variants thereof); and various known antitumor or anticancer agents.

As used herein, the "percent identity (%)" for an amino acid sequence refers to the percentage calculated as follows: after aligning a candidate sequence to a specific amino acid sequence provided herein over a comparison window and introducing gaps, if necessary, to achieve the maximum percent sequence identity, and not considering any conservative substitutions as part of sequence identity, the number of positions within the comparison window at which the candidate sequence contains the same amino acid residue as the specified amino acid sequence is counted and divided by the total number of residues in the comparison window to obtain the percentage. Unless otherwise specified, the comparison window is the full length of the specified amino acid sequence.

As used herein, with respect to the constant domains CH2, CH3, and Fc, the term "wild type" means that the constant domain has the sequence of a native immunoglobulin constant domain, or differs from the native sequence by no more than 1-5 amino acid alterations (preferably no more than 1, 2, 3, 4, or 5 amino acid alterations, wherein the alterations are more preferably conservative amino acid substitutions).

For a polypeptide sequence, "conserved modifications" include substitutions, deletions, or additions to the polypeptide sequence that result in the replacement of an amino acid with a chemically similar amino acid. Tables providing conserved substitutions of functionally similar amino acids are well known in the art. The following eight groups contain amino acids that are conservatively substituted for one another: 1) alanine (A), glycine (G); 2) aspartic acid (D), glutamic acid (E); 3) asparagine (N), glutamine (Q); 4) arginine (R), lysine (K); 5) isoleucine (I), leucine (L), methionine (M), valine (V); 6) phenylalanine (F), tyrosine (Y), tryptophan (W); 7) serine (S), threonine (T); and 8) cysteine (C), methionine (M) (see, e.g., Creighton, Proteins (1984)).

As used herein, the term "host cell" refers to a cell into which an exogenous polynucleotide has been introduced, including progeny of such cell. Host cells include "transformants" and "transformed cells," which encompass the primary transformed cell and progeny derived therefrom. A host cell may be any cell system useful for producing the polypeptides or protein molecules of the present invention, including eukaryotic cells such as mammalian cells, insect cells, and yeast cells; and prokaryotic cells such as *E. coli* cells. Host cells include cultured cells, as well as cells present in transgenic animals, transgenic plants, or cultured plant or animal tissues.

As used herein, the term "expression vector" refers to a vector comprising a recombinant polynucleotide that includes an expression control sequence operably linked to the nucleotide sequence to be expressed. The expression vector contains cis-acting elements sufficient for expression; additional elements for expression may be provided by the host cell or by an in vitro expression system. Expression vectors include all those known in the art, including, but not limited to, cosmides, plasmids (e.g., naked or contained in liposomes), and viruses (e.g., lentiviruses, retroviruses, adenoviruses, and adeno-associated viruses).

As used herein, the term "individual" refers to a mammal. Mammals include, but are not limited to, domesticated animals (e.g., cows, sheep, cats, dogs, and horses), primates (e.g., humans and non-human primates such as monkeys), rabbits, and rodents (e.g., mice and rats). In particular, the individual is a human.

As used herein, the term "treatment" refers to a clinical intervention intended to alter the natural course of the disease in an individual receiving such treatment. Desired therapeutic effects include, but are not limited to, preventing the onset or recurrence of a disease, alleviating symptom, reducing any direct or indirect pathological consequence of the disease, preventing metastasis, slowing the rate of disease progression, improving or mitigating the disease state, and alleviating or improving prognosis.

As used herein, the terms "cancer" and "tumor" are used interchangeably, and refer to or describe a physiological condition in mammals that is typically characterized by unregulated cell growth. Examples of cancer include, but are not limited to, carcinomas, solid tumors, and liquid tumors.

### II. Heterodimeric Fc scaffold

As demonstrated in the Examples, by introduction of the specific Fc mutation combination of the invention, the heterodimeric Fc scaffold according to the present invention yields an Fc heterodimer proportion of greater than 90% after cellular expression and affinity chromatography purification, which is higher than that of the control Fc protein containing only the corresponding knobs-into-holes mutations. After further purification by ion-exchange chromatography, the purity exceeds 98%. In addition, the purified Fc heterodimer exhibits improved thermal stability compared with the control Fc protein, with a CH3-domain Tm greater than 74 °C as determined by DSC.

In a first aspect, therefore, the present disclosure provides a heterodimeric Fc scaffold useful for enhancing the formation of a heteromultimer of interest. The heterodimeric Fc scaffold of the invention greatly increases the yield of the desired heteromultimer product relative to unwanted heteromultimeric and homomultimeric impurities, thereby improving production efficiency and reducing production costs of the heteromultimer, while also improving the thermal stability of the heteromultimer product.

The heterodimeric Fc scaffold of the invention comprises two immunoglobulin Fc regions that are paired and heterodimerized, each comprising a CH3 domain. The heterodimeric Fc scaffold of the invention is characterized in that in addition to knobs-into-holes (KIH) mutations, the CH3 domains further comprise:
(a) in the Fc region comprising the hole mutation, an L351Y mutation; or
(b) in the Fc region comprising the hole mutation, L351Y and D399R mutations, and in the Fc region comprising the knob mutation, a K409D mutation.

For brevity, the mutation combinations described above are sometimes referred to in the present disclosure as "the characteristic CH3 interface mutations of the invention". Likewise, for brevity, the Fc regions comprising the hole mutation and polypeptides comprising the same are referred to as a hole chain; the Fc regions comprising the corresponding knob mutation and polypeptides comprising the same are referred to as a knob chain. Accordingly, with respect to additional mutations introduced into a hole chain, the term "Hole" is placed before the mutation to indicate that the mutation is present in the hole chain; similarly, with respect to additional mutations introduced into a knob chain, the term "Knob" is placed before the mutation to indicate that the mutation is present in the knob chain. For example, Hole L351Y refers to the substitution of a leucine residue (L) with a tyrosine residue (Y) at position 351 in a hole-chain Fc region; and Knob K409D refers to the substitution of a lysine residue (K) with an aspartic acid residue (D) at position 409 in a knob-chain Fc region.

Knobs-into-holes (KIH) modification techniques are known in the art. See, e.g., US7951917B1; US 5,731,168; US 7,695,936; Ridgway et al., Prot Eng 9, 617-621 (1996); and Carter, J Immunol Meth 248, 7-15 (2001). Typically, the technique involves introducing a protrusion ("knob" or knob mutation) at the CH3 domain interface of the Fc region of a first polypeptide, and a corresponding cavity ("hole" or hole mutation) at the CH3 domain interface of the Fc region of a second polypeptide, such that the protrusion can be positioned within the cavity to promote heterodimer formation between the first and second polypeptides and suppress homodimer formation. The protrusion may be generated by replacing an amino acid having a small side chain at the interface of the first polypeptide with an amino acid having a larger side chain (e.g., tyrosine or tryptophan). A complementary cavity of the same or similar size as the protrusion may be created at the interface of the second polypeptide by replacing an amino acid having a large side chain with an amino acid having a smaller side chain (e.g., alanine or threonine). Such a combination of protrusion and cavity mutations that are spatially and dimensionally complementary is referred to in the art as knobs-into-holes (KIH) mutations, wherein the protrusion mutation is termed a knob mutation of the KIH, and the cavity mutation is termed a hole mutation of the KIH.

In a specific embodiment, therefore, the Fc scaffold of the invention comprises KIH mutations, wherein the CH3 domain of one of the first and second Fc regions of the Fc scaffold comprises a knob mutation, and the CH3 domain of the other of the first and second Fc regions comprises a corresponding, complementary hole mutation. In a more specific embodiment, an amino acid substitution is introduced into the CH3 domain of one of the first and second Fc regions (e.g., the first Fc region) of the Fc scaffold, such that one or more amino acid residues are replaced with amino acid residues having a larger side chain, thereby forming a protrusion in the CH3 domain of said Fc region; concurrently, an amino acid substitution is introduced into the CH3 domain of the other of the first and second Fc regions (e.g., the second Fc region) of the Fc scaffold, such that one or more amino acid residues are replaced with amino acid residues having a smaller side chain, thereby forming a cavity in the CH3 domain of said Fc region that is spatially complementary to the aforementioned protrusion. In this manner, the protrusion in the CH3 domain of one Fc region can be accommodated within the cavity in the CH3 domain of the other Fc region. The protrusions and cavities may be generated by modifying nucleic acids encoding the polypeptides, for example, by site-directed mutagenesis, or by peptide synthesis. In some more specific embodiments, the Fc scaffold of the invention comprises KIH mutations wherein the knob mutation is T366W and the hole mutation is Y407V. In other more specific embodiments, the Fc scaffold of the invention comprises KIH mutations wherein the knob mutation is T366W and the hole mutation is T366S-L368A-Y407V.

In some embodiments, the Fc regions of the heterodimeric Fc scaffold of the invention consist of, or consist essentially of, CH3 domains. In such embodiments, the present disclosure thus provides a CH3 heterodimer of the invention.

In some embodiments, the heterodimeric Fc scaffold of the invention, or a heteromultimeric protein of the invention comprising the scaffold, further comprises additional immunoglobulin constant domain(s) in addition to a CH3 domain. For example, the scaffold and/or protein may comprise a CH2 domain and/or a hinge region in an Fc region, and/or a CH1 domain and/or a CL domain in a target-binding domain and linked to a VH or VL domain. In such embodiments, these immunoglobulin constant domains may be of the same or different species origin (but preferably all of human origin) and/or of the same or different immunoglobulin classes or subclasses (but preferably of the same IgG class or IgG subclass). In one embodiment, the Fc scaffold of the invention comprises CH2 and CH3 domains derived from an IgG immunoglobulin (particularly human IgG). In one embodiment, the Fc scaffold of the invention further comprises a hinge region and/or a CH1 domain derived from an IgG immunoglobulin (particularly human IgG). As will be understood by those skilled in the art, reference to the species origin and class of an immunoglobulin constant domain indicates that the constant domain comprises the native amino acid sequence of an immunoglobulin of the indicated species and class, or a variant thereof, wherein amino acid alterations in the variant sequence do not affect the assignment of the species origin, class or subclass of the constant domain. For the purposes of the present invention, the variant sequence comprises typically no more than 10 amino acid residue changes relative to the corresponding native sequence. By way of example, structural features of various IgG subclasses are described in Gestur Vidarsson et al., IgG Subclasses and Allotypes: From Structure to Effector Functions, Front. Immunol. (2014) 5:520, doi:10.3389/fimmu.2014.00520, which is incorporated herein by reference in its entirety. In some embodiments, the immunoglobulin constant domains comprised in the heterodimeric Fc scaffold or heteromultimeric protein of the invention are an IgG immunoglobulin constant domain. In some embodiments, the constant domains are independently selected from IgG subclasses, including IgG1, IgG2, IgG3, and IgG4, but are preferably all IgG1 or all IgG4. In some embodiments, the constant domain is an IgG immunoglobulin constant domain of human origin.

In some specific embodiments, the heterodimeric Fc scaffold of the invention comprises an IgG Fc region. In some more specific embodiments, the Fc region is an IgG1 Fc region. In other specific embodiments, the Fc region is an IgG4 Fc region.

In some embodiments, the Fc regions of the heterodimeric Fc scaffold of the invention have an effector function. In some embodiments, the effector function is Fcγ receptor binding and/or FcRn receptor binding. In some embodiments, the Fcγ receptor is human FcγRIIa, FcγRI, and/or FcγRIIIa, particularly FcγRI. In some embodiments, the heterodimeric Fc scaffold of the invention (or a binding molecule comprising the heterodimeric Fc scaffold) exhibits an Fcγ receptor binding affinity that is not less than 60%, for example 50%, 40%, 30%, 20%, or 10%, relative to a native IgG1 dimeric Fc domain (or a binding molecule comprising a native IgG1 dimeric Fc domain). In some embodiments, the Fcγ receptor is FcγRI. In some embodiments, for FcRn receptor binding, the heterodimeric Fc scaffold of the invention (or a binding molecule comprising the heterodimeric Fc scaffold) exhibits an FcRn binding affinity that is substantially similar to that of a native IgG1 dimeric Fc domain (or a binding molecule comprising a native IgG1 dimeric Fc domain), i.e., more than about 70%, particularly more than about 80%, and more particularly more than about 90%, of the FcRn binding affinity of the native IgG1 dimeric Fc domain.

An Fc domain may confer favorable pharmacokinetic properties on a heteromultimeric protein comprising the same (e.g., a bispecific antibody), including prolonged serum half-life, favorable accumulation in target tissues, and a favorable tissue-to-blood partition ratio. However, an Fc domain may also direct a heteromultimeric protein comprising the same (e.g., a bispecific antibody) to cells expressing Fcγ receptors more frequently than desired cells and/or tissues harboring the target of interest. Furthermore, activation of Fcγ receptor signaling pathways may lead to cytokine release, resulting in adverse side effects upon systemic administration of Fc domain-containing heteromultimeric proteins. Accordingly, in some embodiments, in addition to the characteristic CH3 interface mutations of the invention, the heterodimeric Fc scaffold of the invention may comprise or not comprise additional mutations as needed, in order to maintain or modulate one or more effector functions thereof. For example, it is known that the upper CH2 domain and hinge region of an antibody participate in multiple effector functions, such as Fcγ receptor binding and ADCC; and certain CH2 and CH3 interface residues of an antibody are involved in FcRn receptor binding. In some instances, the Fc region for use in the heterodimeric scaffold of the invention may comprise additional mutations introduced into the CH2 domain and/or hinge region (if present) to alter (reduce or enhance) one or more selected effector functions, such as the binding affinity of the Fc region for an Fcγ receptor and/or FcRn. Such amino acid modifications are known in the art and include, but are not limited to, amino acid substitutions at one or more positions selected from E233, L234, L235, N297, P329, and P331, and more particularly substitutions at one or more amino acids selected from E233P, L234A, L235A, L235E, N297A, N297D, and P331S. In some instances, where the Fc scaffold comprises a human IgG1 Fc region, binding of the Fc scaffold to an Fcγ receptor is substantially reduced by inclusion in the Fc region of L234A-L235A mutations, L234A-L235A-P329G mutations, and/or N297A mutation.

Exemplary Fc scaffold sequences according to the present invention are provided herein. In some embodiments, the heterodimeric Fc scaffold of the invention comprises a first polypeptide and a second polypeptide, wherein the first polypeptide comprises a first Fc region and the second polypeptide comprises a second Fc region, wherein:
(a) relative to a wild-type CH3 domain, each of the CH3 domains of the first and second Fc regions comprise 0-3 amino acid residue changes, in addition to the characteristic CH3 interface mutations of the invention as defined above; preferably, the wild-type CH3 domain is a CH3 domain of a native IgG immunoglobulin (particularly IgG1 or IgG4, preferably human IgG1), and more preferably comprises the amino acid sequence of SEQ ID NO: 19 or 20;
(b) the first and second Fc regions comprise a CH2 domain of a native IgG immunoglobulin (particularly IgG1 or IgG4, preferably human IgG1), or a CH2 domain having the amino acid sequence of SEQ ID NO: 21 or comprising 1-5 amino acid residue changes relative thereto;
(c) the Fc region comprising the knob mutation comprises the amino acid sequence of SEQ ID NO: 1 or 2, or an amino acid sequence having at least 95%, 96%, 97%, 98%, or 99% sequence identity thereto;
(d) the Fc region comprising the hole mutation comprises the amino acid sequence of SEQ ID NO: 7 or 8, or an amino acid sequence having at least 95%, 96%, 97%, 98%, or 99% sequence identity thereto; and/or
(e) the Fc scaffold does not form a non-naturally occurring disulfide bond between the first Fc region and the second Fc region.

In some preferred embodiments, the Fc region comprising the knob mutation comprises a CH3 domain having the amino acid sequence as shown in SEQ ID NO: 1 or 2, or an amino acid sequence having at least 95% identity thereto; and the Fc region comprising the hole mutation comprises a CH3 domain having the amino acid sequence as shown in SEQ ID NO: 7 or 8, or an amino acid sequence having at least 95% identity thereto. In some embodiments, the Fc region comprising the knob mutation comprises a CH3 domain having the amino acid sequence as shown in SEQ ID NO: 1 or 2; and the Fc region comprising the hole mutation comprises a CH3 domain having the amino acid sequence as shown in SEQ ID NO: 7 or 8. In some embodiments, the Fc region comprising the knob mutation comprises a CH3 domain having the amino acid sequence as shown in SEQ ID NO: 1, and the Fc region comprising the hole mutation comprises a CH3 domain having the amino acid sequence shown in SEQ ID NO: 7. In some embodiments, the Fc region comprising the knob mutation comprises a CH3 domain having the amino acid sequence as shown in SEQ ID NO: 2, and the Fc region comprising the hole mutation comprises a CH3 domain having the amino acid sequence as shown in SEQ ID NO: 8. In any of the foregoing embodiments, the Fc region comprising the knob mutation may be the first Fc region and the Fc region comprising the hole mutation may be the second Fc region, or vice versa.

In some embodiments, the heterodimeric Fc scaffold of the invention does not comprise non-naturally occurring disulfide bonds introduced at the CH3-domain interface between the first and second Fc regions, yet retains favorable thermal stability. For example, the CH3-domain Tm is 70°C or higher as measured by DSC.

In some embodiments, the heterodimeric Fc scaffold of the invention exhibits improved thermal stability relative to a corresponding control Fc scaffold bearing the same KIH mutations. As used herein, the term "corresponding control Fc scaffold" refers to a heterodimeric Fc scaffold that, relative to the heterodimeric Fc scaffold of the invention being compared, is identical in amino acid sequence (including KIH mutations), except that it comprises native (unmutated) residues at positions L351, D366, and K409.

In some embodiments, the heterodimeric Fc scaffold of the invention exhibits a purity comparable to that of a corresponding control Fc scaffold comprising the same KIH mutations. In some embodiments, following expression, assembly, and purification from host cells under identical conditions, the purity of the heterodimeric Fc scaffold of the invention is at least 90%, 95%, 100%, 110%, or more relative to the purity of the corresponding control Fc scaffold. Preferably, the heterodimeric Fc scaffold has a purity greater than 90%, as determined by SEC-HPLC.

### III. Heteromultimeric protein

In a second aspect, the present disclosure provides a heteromultimeric protein comprising a CH3 heterodimer of the invention, or comprising a heterodimeric Fc scaffold of the invention.

In some embodiments, the heteromultimeric protein according to the invention comprises at least a first polypeptide and a second polypeptide, wherein the first polypeptide comprises a first CH3 domain and the second polypeptide comprises a second CH3 domain, and wherein the first CH3 domain and the second CH3 domain interact at an interface to form a CH3 heterodimer of the invention.

In some embodiments, the heteromultimeric protein according to the invention comprises at least a first polypeptide and a second polypeptide, wherein the first polypeptide comprises a first Fc region and the second polypeptide comprises a second Fc region, and wherein the first Fc region and the second Fc region interact at an interface to form an Fc scaffold of the invention.

In some embodiments, the heteromultimeric protein is a mono- or multi-specific binding protein comprising at least one target-binding domain, wherein the at least one target-binding domain is linked to a CH3 heterodimer of the invention or to a heterodimeric Fc scaffold of the invention. In some embodiments, the heteromultimeric protein according to the invention comprises more than one of the Fc scaffold. In some embodiments, the heteromultimeric protein according to the invention comprises only one of the Fc scaffold. Such mono- or multi-specific binding proteins comprising the Fc scaffold of the invention are also referred to herein as "Fc scaffold-based binding proteins."

### Fc scaffold-based binding proteins

In some aspects, therefore, the present disclosure provides a binding protein based on a heterodimeric Fc scaffold of the invention. In some embodiments, the binding protein according to the present invention comprises at least one target-binding domain linked to the Fc scaffold.

### Type of Target-Binding Domain

The type of target-binding domain used in a binding protein of the invention is not particularly limited, provided that it is capable of binding to a target of interest. As is known in the art, target-binding domains may be derived from various binding partners, including non-immunoglobulin-based binding pairs (e.g., receptor/ligand pairs and enzyme/substrate pairs) and immunoglobulin-based binding pairs (e.g., antigen/antibody pairs and antibody/anti-idiotype antibody pairs). Where the target-binding domain is derived from an immunoglobulin-based binding pair, the binding protein comprising the domain may be an antibody, such as a monospecific or multispecific antibody. Where the target-binding domain is derived from a non-immunoglobulin-based binding pair, the binding protein comprising the domain may be an immunoadhesin. Furthermore, the present disclosure also contemplates binding proteins that incorporate binding domains derived from both categories of binding pairs; such antibody-immunoadhesin chimeric proteins are sometimes also referred to herein as antibodies.

In some embodiments, the binding protein according to the invention comprises at least one antigen-binding domain. Antigens that bind to an antigen-binding domain of the invention may be selected from, for example, but not limited to: tumor-associated antigens, immune checkpoint molecules, angiogenic factors, or combinations thereof. Tumor-associated antigens include, but are not limited to, MUC1, BCMA, CLDN18.2, HER2, BRAF, EGFR, CD20, CD38, FOLR1, and CD52. Immune checkpoint molecules include, but are not limited to, PD-L1, PD-1, PD-L2, CTLA-4, B7-H3, TIM-3, LAG-3, VISTA, ICOS, 4-1BB, OX40, GITR, and CD40. Angiogenic factors include, but are not limited to, basic FGF, HGF, sTie-2, sVEGFR-1, sVEGFR-2, EGF, IL-6, IL-8, PLGF, VEGF, PDGF-BB, ANG1, ANG2, SDF-1α, MDC, galectin, TSP-1, endocan, eNOS, and HIF-1α. In some embodiments, the antigen is selected from tumor-associated antigens, such as EGFR and HER2.

In some embodiments, the binding protein according to the invention comprises at least one non-immunoglobulin-based binding domain.

In some embodiments, examples of non-immunoglobulin-based binding domains that may be used include, but are not limited to, ligand-binding domains derived from the following receptor proteins: 4-1BB; adrenocorticotropic hormone receptor; activin receptor; BLTR (leukotriene B4 receptor); BMP receptor; C3a receptor; C5a receptor; chemokine receptor; cytokine receptor; growth hormone receptor; BTLA; interferon-α receptor; interferon-β receptor; interferon-γ receptor; type I IL-1 receptor; type II IL-1 receptor; IL-10 receptor; IL-11 receptor; IL-12 receptor; BCMA; TACI; BAFF receptor; immunomodulatory signaling receptor CD72; Kaposi's sarcoma-associated herpesvirus GPCR; lipoxygenin A4 receptor; lymphotoxin β receptor; RON receptor; SCF receptor; somatostatin receptor; T1/ST2; TGF-β receptor; tumor necrosis factor receptor; TNFRSF19; erythropoietin receptor; leukemia inhibitory factor receptor; and C-kit receptor.

In some embodiments, examples of non-immunoglobulin-based binding domains that may be used include, but are not limited to, receptor-binding domains derived from the following ligands, or the ligands themselves: α-MSH; 9E3/cCAF; adrenocorticotropic hormone; activin; AK155; Angiostatin; Apo2L/TRAIL; BLR1 ligand/BCA-1/BLC/CXCL13; calcitonin gene-related peptide; CD27 ligand; CD30 ligand; CD40 ligand; endorphin; endostatin; erythropoietin; Fas ligand; Flt-3 ligand; G-CSF; GCP-2/CXCL6; GM-CSF; various cytokines, such as IFNα, IFNβ; interferon γ; IL-1α; IL-1β; IL-10; IL-11; IL-12; IL-13; IL-15; IL-16; IL-2; IL-27; IL-3; IL-4; IL-5; IL-6; IL-7; IL-8/CXCL8; IL-9; somatostatin; stem cell factor; substance P; TARC/CCL17; TCA3/mouse CCL1; TECK/CCL25; TGFβ; thrombopoietin; TNFα.

In some embodiments, the non-immunoglobulin-based binding domain is derived from a protein selected from: VEGF receptor; TNF receptor; IL-1; lymphocyte function-associated antigen 3 (LFA-3/CD58); lymphotoxin β receptor (LTBR); CTLA-4; IL-12; activin receptor; TACI; BR3; T cell receptor; CD4; L-selectin; homing receptor; CD44; NP receptor; interferon-γ receptor; 4-1BB and IgE receptor. In some other embodiments, the non-immunoglobulin-based binding domain is derived from a receptor-binding domain of a ligand selected from IL-1 and IL-12, or is the ligand itself. Additional examples of non-immunoglobulin-based binding domains that may be used are described, for example, in US 7,951,917 B1 and US 2022/0275048 A1.

### Target-Binding Domain Linkage

In a binding protein of the invention, the attachment position(s) of a target-binding domain on the heterodimeric Fc scaffold is/are not particularly limited. In some embodiments, the at least one target-binding domain, independently of one another, is linked to the N-terminus or the C-terminus of the first and/or second polypeptide of the Fc scaffold. In some embodiments, at least two, a plurality of, or all of the at least one target-binding domain are linked to each other, optionally via a peptide linker, and then linked to the heterodimeric Fc scaffold of the invention. In some other embodiments, at least two, a plurality of, or all of the at least one target-binding domain are separately linked to different termini of the Fc scaffold, optionally via a peptide linker. For example, where the binding protein comprises two target-binding domains, one domain may be linked to the N-terminus of the first polypeptide of the Fc scaffold, while the other domain may be linked to a different terminus of the Fc scaffold, such as the C-terminus of the first polypeptide, the N-terminus of the second polypeptide, or the C-terminus of the second polypeptide. Accordingly, at least one, two, three, or all of the four termini of the heterodimeric Fc scaffold (i.e., the N- and C-termini of the first and second polypeptides) may each be linked to at least one (and preferably one or two) target-binding domain.

The at least one target-binding domain comprised in the binding protein may be the same as or different from each other. In one embodiment, at least two, a plurality of, or all of the at least one target-binding domain are identical. In some other embodiments, at least two, a plurality of, or all of the at least one target-binding domain are different from each other.

In some embodiments, the binding protein is monospecific and comprises one or more binding domain(s) for that specificity. In some embodiments, the binding protein is multispecific and preferably comprises one or more binding domain(s) for each specificity.

In some embodiments, the at least one target-binding domain is independently selected from the group consisting of Fab, VHH, scFv, scFab, and crossFab antigen-binding domains. When the binding domain is Fab or crossFab, it may be linked, optionally via a peptide linker, to another binding domain of the binding protein or to the Fc scaffold (e.g., to the N-terminus of the first or second polypeptide of the heterodimeric Fc scaffold of the invention) through the C-terminus of the VH-containing chain or the C-terminus of the VL-containing chain.

In some embodiments, at least one of the at least one target-binding domain is a VHH domain. In some other embodiments, at least one of the at least one target-binding domain is a Fab domain. In some other embodiments, at least one of the at least one target-binding domain is a VHH domain and at least one of the at least one target-binding domain is a Fab domain. In some other embodiments, at least two, a plurality of, or all of the at least one target-binding domains are Fab domains, and the Fab domains comprise identical or different VL-CL light chains.

In some embodiments, the binding protein is a mono-specific, monovalent protein comprising a VHH domain, and preferably, the VHH domain is linked to the N-terminus of the first or second polypeptide of the Fc scaffold. In some embodiments, the binding protein is a bispecific, bivalent protein comprising VHH and/or Fab binding domains. In some embodiments, the binding protein is a bispecific antibody in an IgG format, comprising first and second Fab domains and an Fc scaffold. Preferably, the first and second Fab domains are linked to the N-termini of the first and second polypeptides of the Fc scaffold, respectively, via the C-terminus of their VH-containing chains. In some embodiments, the binding protein is a bispecific antibody in an IgG-like format comprising a VHH and a Fab, wherein the antibody consists of: a heavy chain comprising a VHH-Fc domain; a heavy chain comprising a VH-Fc domain; and a light chain comprising a VL-CL domain.

The term "link," as used herein with respect to distinct components within a scaffold or binding protein, means that the components are fused or conjugated, either directly or via a suitable linker. Peptide linkers suitable for this purpose are generally short, flexible amino acid polypeptides, and typically range from 1 to 50 amino acid residues in length, although not limited thereto. In some instances, any amino acid chain located between two components being linked may be regarded as a peptide linker, provided that it does not impair the intended function of said components. Accordingly, in some embodiments, the present disclosure envisages heterodimeric Fc scaffolds of the invention, or CH3 heterodimers of the invention, linked to a polypeptide of interest (e.g., a target-binding domain as described herein, or another functional polypeptide or protein domain) via a peptide linker.

Those skilled in the art can readily determine a linker sequence suitable for use in a heteromultimeric protein of the invention, based on the components and the sites to be linked. In some embodiments, the linker is used to link the Fc scaffold of the invention to an antigen-binding domain, and/or to link two antigen-binding domains. In some embodiments, the linker that may be used is a flexible linker peptide of 5-50 amino acids, preferably comprising glycine (G), and/or serine (S), and/or threonine (T) residues. In some embodiments, the linker has a length of 5-30 amino acids, for example 8, 10, 15, 20, 25, or 30 amino acids, or a length between any two of these integers. In some embodiments, the linker comprises the amino acid sequence (G₄S)ₙ, where n is an integer of 1 or more, for example 2, 3, 4, 5, 6, or 7. In a preferred embodiment, the linker consists of the amino acid sequence (G₄S)₂. In some other embodiments, the linker is an immunoglobulin-derived hinge region or a derivative thereof. Additional linkers that may be used include, but are not limited to, the following amino acid sequences: (Gly₃Ser)₂ (SEQ ID NO: 22), (Gly₄Ser)₂ (SEQ ID NO:12), (Gly₃Ser)₃ (SEQ ID NO: 24), (Gly₄Ser)₃ (SEQ ID NO: 25), (Gly₃Ser)₄ (SEQ ID NO: 26), (Gly₄Ser)₄ (SEQ ID NO: 27), (Gly₃Ser)₅ (SEQ ID NO: 28), (Gly₄Ser)₅ (SEQ ID NO: 29), (Gly₃Ser)₆ (SEQ ID NO: 30), (Gly₄Ser)₆ (SEQ ID NO: 31), GGG (SEQ ID NO: 32), DGGGS (SEQ ID NO: 33), TGEKP (SEQ ID NO: 34), GGRR (SEQ ID NO: 35), EGKSSGSGSESKVD (SEQ ID NO: 36), KESGSVSSEQLAQFRSLD (SEQ ID NO: 37), GGRRGGGS (SEQ ID NO: 38), LRQRDGERP (SEQ ID NO: 39), LRQKDGGGSERP(SEQ ID NO: 40), and GSTSGSGKPGSGEGSTKG (SEQ ID NO:23). Alternatively, if desired, suitable flexible linker peptides can be rationally designed using computer programs to simulate the three-dimensional structure of proteins and peptides, or via phage display methods.

In a preferred embodiment, a peptide linker having the sequence shown in SEQ ID NO: 12 is used to link an antigen-binding domain, such as a VHH or a Fab, to the N-terminus of the Fc scaffold.

In some instances, chemically synthesized linkers may be used to link a binding domain to the Fc scaffold. In such circumstances, such "linkage" is also be referred to as "conjugation". Examples of chemically synthesized linkers include: N-hydroxysuccinimide (NHS), Disuccinimidyl suberate (DSS), Bis(sulfosuccinimidyl) suberate (BS3), Dithiobis(succinimidyl propionate) (DSP), Dithiobis(sulfosuccinimidyl propionate) (DTSSP), Ethylene glycol bis(succinimidyl succinate) (EGS), Ethylene glycol bis(sulfosuccinimidyl succinate) (Sulfo-EGS), Disuccinimidyl tartrate (DST), Disulfosuccinimidyl tartrate (Sulfo-DST), bis[2-(succinimidooxycarbonyloxy)ethyl]sulfone, (BSOCOES), bis[2-(sulfosuccinimidooxycarbonyloxy)ethyl]sulfone (Sulfo-BSOCOES), etc.

When multiple linkers are used in a heteromultimeric protein of the present invention, it will be understood that the linkers may be identical or different.

In some instances, chemically synthesized linkers may also be used to conjugate a chemically synthesized therapeutic agent (e.g., a small-molecule toxin) or a diagnostic agent to the heteromultimeric protein of the invention (e.g., a bispecific antibody), for example to a glycan chain of the CH2 domain. Such conjugates are also within the scope of the present invention.

### Exemplary heteromultimeric protein

In some embodiments, the heteromultimeric protein of the invention is a multi-specific binding protein, particularly a bispecific antibody, that binds to different antigenic epitopes. Bispecific antibodies that specifically bind two distinct antigens or epitopes have broad clinical potential. They may be used as targeted agents for *in vitro* and *in vivo* immunodiagnostic and therapeutic applications, as well as for diagnostic immunoassays. See, e.g., WO 9850431A2. Bispecific antibodies may be used in *in vitro* assays to assess functional properties of cell-surface molecules and to measure cytotoxicity mediated by various Fc receptors (Fanger et al., Crit. Rev. Immunol. 12:101-124, 1992); in enzyme-linked immunosorbent assays (Nolan et al., Biochim. Biophys. Acta 1040:1-11, 1990; Hammerling et al., J. Exp. Med. 128:1461-1473, 1968); and in the immunodiagnosis of diseases, including cancer, *in vitro* or *in vivo* (Songsivilai et al., Clin. Exp. Immunol. 79:315, 1990). For diagnostic applications, one arm of a BsAb can bind an antigen (e.g., a tumor-associated antigen) on the surface of afflicted tissues or cells, while the other arm can bind a detectable label, such as a chelator configured to tightly bind a radionuclide. For therapeutic applications, bispecific antibodies may be used to direct a patient's cellular immune defensive mechanisms specifically toward afflicted tissues or cells, such as tumor cells, or toward pathogens. To this end, one arm of a BsAb can bind an antigen on the surface of immune cells (e.g., T cells or NK cells), and the other arm can bind an antigen on the surface of afflicted tissues or cells.

In some embodiments, a multispecific antibody according to the present invention is a heterodimer consisting of, or consisting essentially of, a heterodimeric Fc scaffold of the invention linked to at least one antigen-binding domain, and having a format selected from the following:
- (antigen-binding domain)n-Fc/(antigen-binding domain)n-Fc,
- (antigen-binding domain)n-Fc-(antigen-binding domain)m/(antigen-binding domain)n-Fc,
- (antigen-binding domain)n-Fc-(antigen-binding domain)m/(antigen-binding domain)n-Fc-(antigen-binding domain)m,
wherein:
each of n and m is an integer independently selected from 0, 1, and 2;
each antigen-binding domain is independently selected from: an Fv, an scFv, an Fab, an scFab, a crossFab, a VHH, a ligand, and a ligand-binding domain of a receptor;
the symbol "-Fc" indicates linkage at the N-terminus of the Fc;
the symbol "Fc-" indicates linkage at the C-terminus of the Fc;
wherein the two monomers of the heterodimer are separated by the symbol "/"; and
wherein one of the two Fcs is a knob chain and the other is a hole chain.

In some preferred embodiments, n is 1 and m is 0. In some preferred embodiments, n is 0 and m is 1. In some embodiments, n is 1 and m is 1. In some preferred embodiments, each antigen-binding domain is independently selected from an scFv, a VHH, and an Fab.

In some specific embodiments, the multispecific heterodimeric protein according to the present invention is bispecific, and is in an IgG format or an IgG-like format.

In some specific embodiments, the multispecific heterodimeric protein according to the present invention is a bispecific antibody having a format selected from the following:
- scFv-Fc/scFv-Fc, wherein scFv domains of two different specificities are respectively linked to the N-termini of the Fc scaffold of the invention;
- Fab-Fc/Fab-Fc, wherein Fab domains of two different specificities are respectively linked to the N-termini of the Fc scaffold of the invention;
- VHH-Fc/VHH-Fc, wherein VHH domains of two different specificities are respectively linked to the N-termini of the Fc scaffold of the invention;
- scFv-Fc/Fab-Fc, wherein an scFv domain and a Fab domain of two different specificities are respectively linked to the N-termini of the Fc scaffold of the invention;
- scFv/VHH-Fc, wherein an scFv domain and a VHH domain of two different specificities are respectively linked to the N-termini of the Fc scaffold of the invention;
- VHH/Fab-Fc, wherein a VHH domain and a Fab domain of two different specificities are respectively linked to the N-termini of the Fc scaffold of the invention.

In embodiments in which the heteromultimeric protein of the invention comprises two or more Fab domains with different specificities (e.g., a bispecific antibody having the Fab-Fc/Fab-Fc format), a common light chain may be used to suppress a light-chain mispairing. See, e.g., US7951917B. Alternatively, the first Fab-Fc half-antibody and the second Fab-Fc half-antibody may be produced separately and then assembled *in vitro* to form the desired heterodimer by adding a suitable reducing agent to the mixture thereof.

In some embodiments, the heteromultimeric protein of the invention is a bispecific antibody comprising a VHH domain and a Fab domain, which bind to different antigens, respectively. In some embodiments, the bispecific antibody binds to HSA and B7-H3. In some embodiments, the VHH domain (i) comprises the CDR1, CDR2, and CDR3 sequences of the VHH amino acid sequence shown in SEQ ID NO: 11; (ii) comprises the amino acid sequence shown in SEQ ID NO: 11; or (iii) comprises an amino acid sequence having at least 95% sequence identity to SEQ ID NO: 11. In some embodiments, the Fab domain comprises a VH and a VL, wherein the VH (i) comprises the CDR1, CDR2, and CDR3 sequences of the VH amino acid sequence shown in SEQ ID NO: 13; (ii) comprises the amino acid sequence shown in SEQ ID NO: 13; or (iii) comprises an amino acid sequence having at least 95% sequence identity to SEQ ID NO: 13; and wherein the VL (i) comprises the CDR1, CDR2, and CDR3 sequences of the VL amino acid sequence shown in SEQ ID NO: 14; (ii) comprises the amino acid sequence shown in SEQ ID NO: 14; or (iii) comprises an amino acid sequence having at least 95% sequence identity to SEQ ID NO: 14. In some embodiments, the VHH domain comprises the amino acid sequence shown in SEQ ID NO: 11, and the Fab domain comprises a VH having the amino acid sequence shown in SEQ ID NO: 13 and a VL having the amino acid sequence shown in SEQ ID NO: 14. In some embodiments, the bispecific antibody is bivalent. In some embodiments, the VHH domain and the Fab domain are linked to the N-termini of the first and second peptides, respectively, of the heterodimeric Fc scaffold of the invention.

In some embodiments, the binding protein of the present invention is a bispecific antibody comprising a first Fab domain and a second Fab domain, which bind to different antigens, respectively. In some embodiments, the bispecific antibody binds to EGFR and HER2. In some embodiments, the first Fab domain comprises a VH and a VL, wherein the VH (i) comprises the CDR1, CDR2, and CDR3 sequences of the VH amino acid sequence shown in SEQ ID NO: 15; (ii) comprises the amino acid sequence shown in SEQ ID NO: 15; or (iii) comprises an amino acid sequence having at least 95% sequence identity to SEQ ID NO: 15; and wherein the VL (i) comprises the CDR1, CDR2, and CDR3 sequences of the VL amino acid sequence shown in SEQ ID NO: 16; (ii) comprises the amino acid sequence shown in SEQ ID NO: 16; or (iii) comprises an amino acid sequence having at least 95% sequence identity to SEQ ID NO: 16. In some embodiments, the second Fab domain comprises a VH and a VL, wherein the VH (i) comprises the CDR1, CDR2, and CDR3 sequences of the VH amino acid sequence shown in SEQ ID NO: 17; (ii) comprises the amino acid sequence shown in SEQ ID NO: 17; or (iii) comprises an amino acid sequence having at least 95% sequence identity to SEQ ID NO: 17; and wherein the VL (i) comprises the CDR1, CDR2, and CDR3 sequences of the VL amino acid sequence shown in SEQ ID NO: 18; (ii) comprises the amino acid sequence shown in SEQ ID NO: 18; or (iii) comprises an amino acid sequence having at least 95% sequence identity to SEQ ID NO: 18. In some embodiments, the Fab domain comprises a VH having the amino acid sequence shown in SEQ ID NO: 15 and a VL having the amino acid sequence shown in SEQ ID NO: 16; and the Fab domain comprises a VH having the amino acid sequence shown in SEQ ID NO: 17 and a VL having the amino acid sequence shown in SEQ ID NO: 18. In some embodiments, the bispecific antibody is bivalent. In some embodiments, the first Fab domain and the second Fab domain are linked to the N-termini of the first and second peptides, respectively, of the heterodimeric Fc scaffold of the invention.

### IV. Polynucleotides, Vectors, and Host Cells

In some aspects, the present disclosure provides one or more polynucleotides encoding a CH3 heterodimer, a heterodimeric Fc scaffold, a heteromultimeric protein, or a binding protein according to the invention; and one or more vectors comprising the one or more polynucleotides, wherein the one or more polynucleotides may be present in a single vector or in multiple separate vectors. In some further aspects, the present disclosure provides host cells comprising the one or more polynucleotides or the one or more vectors. As appropriate, a single vector or multiple vectors comprising the one or more polynucleotides may be introduced into the same host cell to express the desired product in that host cell; alternatively, a single vector or multiple vectors comprising the one or more polynucleotides may be introduced separately into different host cells to express intermediates comprising individual chains or combinations of chains (e.g., different protein monomers) of the heteromultimeric protein of the invention in different host cells, and the intermediates may then be combined under conditions suitable for assembly of the heteromultimeric protein of the invention, thereby producing the heteromultimeric protein.

There are no particular limitations on the expression vectors that may be used in the present disclosure, including, but not limited to, viral vectors, plasmids, cosmids, λ bacteriophages, and yeast artificial chromosomes (YACs).

There are also no particular limitations on the host cells that may be used in the present invention. Suitable host cells include prokaryotic microorganisms (e.g., *Escherichia coli*), eukaryotic microorganisms (e.g., filamentous fungi or yeast), and other eukaryotic cells, such as mammalian cells and insect cells. Examples of useful mammalian host cell lines include SV40-transformed monkey kidney CV1 cells (COS-7), human embryonic kidney cells (HEK293 or 293F), baby hamster kidney cells (BHK), monkey kidney cells (CV1), African green monkey kidney cells (VERO-76), human cervical carcinoma cells (HeLa), Canine kidney cells (MDCK), Buffalo rat liver cells (BRL 3A), human lung cells (W138), human hepatic cells (HepG2), CHO cells, NSO cells, and myeloma cell lines such as Y0, NS0, P3X63, and Sp2/0. In some embodiments, mammalian cell lines suitable for suspension culture are used. In some preferred embodiments, the host cell is a CHO cell or an HEK293 cell.

### V.Production and Purification of Heteromultimeric Proteins of the Invention

In another aspect, the present disclosure provides methods for producing a heteromultimeric protein of the invention. To produce the heteromultimeric protein, the polypeptide chains thereof may be obtained, for example, by solid peptide synthesis (e.g., Merrifield solid-phase synthesis) or by recombinant production, and assembled under suitable conditions. As described herein, in recombinant production, assembly may occur in a host cell expressing the heteromultimeric protein; alternatively, when needed, the heteromultimeric protein may be assembled *in vitro* after harvesting the intermediate polypeptide chains that are expressed from host cells.

In one embodiment, therefore, the present invention provides a method for producing the heteromultimeric protein of the invention, the method comprising: culturing a host cell comprising a nucleic acid encoding the polypeptide chains of the heteromultimeric protein under conditions suitable for expression of the polypeptide chains; and assembling the polypeptide chains under conditions suitable to form the heteromultimeric protein.

In some embodiments, the method comprises: culturing a host cell comprising a nucleic acid encoding the polypeptide chains of the heteromultimeric protein under conditions suitable for expressing said polypeptide chains; and recovering the heteromultimeric protein produced by the host cell from the culture. Preferably, after Protein A affinity chromatography of the recovered product, the heteromultimeric protein of the invention exhibits a purity greater than 80%, preferably greater than 85%, and more preferably greater than 90%, as determined by SEC-HPLC.

In some other embodiments, the method comprises: culturing a host cell comprising a nucleic acid encoding the polypeptide chains of the heteromultimeric protein under conditions suitable for expression of the polypeptide chains; recovering intermediates produced by the host cell from the cell culture; combining the intermediates under conditions suitable for assembly of the heteromultimeric protein; and recovering the resulting heteromultimeric protein. In embodiments involving heterodimeric proteins, monomers comprising knob chains and monomers comprising hole chains are expressed separately in different host cells, and the heterodimeric protein is produced by combining the monomers under conditions suitable for assembly and is recovered from the mixture of the monomers. Preferably, after ion-exchange chromatography of the recovered product, the heteromultimeric protein of the invention exhibits a purity of greater than 80%, preferably greater than 85%, and more preferably greater than 90%, as determined by SEC-HPLC.

The antibodies prepared by the methods described herein can be purified using known existing techniques such as high-performance liquid chromatography (HPLC), ion-exchange chromatography, gel electrophoresis, affinity chromatography, and size exclusion chromatography. After purification, the purity of the heteromultimeric protein of the invention can be determined using any of a variety of well-known analytical methods, including size exclusion chromatography, gel electrophoresis, and HPLC. The physical/chemical properties and/or biological activity of the heteromultimeric protein provided herein can be identified, screened, or characterized using various assays known in the art.

### VI. Compositions and Uses

The present disclosure further relates to a composition comprising a heteromultimeric proteins of the invention (e.g., a binding protein of the invention). In embodiments in which the composition is a pharmaceutical composition, the composition further comprises a pharmaceutically acceptable carrier. As used herein, the term "pharmaceutically acceptable carrier" includes any and all physiologically compatible solvents, dispersion media, isotonicity agents, and absorption-delaying agents, and the like.

As used herein, a pharmaceutical composition generally refers to a medicament for the treatment or prevention of a disease, or for detection or diagnosis of a disease. In some preferred embodiments involving a pharmaceutical composition of the invention, a heteromultimeric protein of the invention is a bispecific antibody, e.g., one having at least one specificity for a disease-associated antigen. In some embodiments, the heteromultimeric protein of the invention is the sole active ingredient in the pharmaceutical composition. In other embodiments, the pharmaceutical composition comprises the heteromultimeric protein described herein in combination with one or more additional therapeutic agents.

The pharmaceutical composition of the invention may be formulated using methods well known to those skilled in the art. Pharmaceutically acceptable carriers or media, such as sterile water or saline, vegetable oils, emulsifiers, suspending agents, surfactants, stabilizers, flavoring agents, excipients, solvents, preservatives, binders, and the like, may be combined as appropriate with the heteromultimeric protein of the invention to provide a unit dosage form suitable for administration. For example, a pharmaceutically acceptable carrier may be used to prepare a sterile solution or a sterile suspension for parenteral administration by injection. The amount of the active ingredient in the formulation may be selected such that administration to an individual provides an effective amount within a predetermined range.

The pharmaceutical composition of the invention is suitable for a variety of routes of administration, including, but not limited to, intravenous, intramuscular, subcutaneous, parenteral, rectal, spinal, and epidermal administration (e.g., by injection or infusion). Accordingly, the pharmaceutical composition may be formulated into a dosage form appropriate for the intended route of administration, such as injectable, intranasal, pulmonary, or transdermal dosage forms. For injectable dosage forms, routes of administration include, but are not limited to, systemic or local administration, such as intravenous, intramuscular, intraperitoneal, subcutaneous, or intratumoral injection. The specific dosing regimen of the pharmaceutical composition may be selected as appropriate based on the subject's age and symptoms.

Furthermore, a detection or diagnostic kit comprising the heteromultimeric protein described herein is also within the scope of the present disclosure. The kit may further comprise one or more additional components, for example: instructions for use; additional reagents, such as labeling reagents or conjugation reagents; a pharmaceutically acceptable carrier; and a device or other material for administration to a subject.

The following Examples are provided to aid the understanding of the present invention. They are not intended, and should not be construed, to limit the scope of the invention in any way.

### Examples

### Example 1: Designing sequences of candidate Fc mutation combinations

Fc heterodimer crystal structures of the knobs-into-holes (KIH) model (e.g., 4NQS and 5ID8) were retrieved from the Protein Data Bank (www.pdb.org) and compared with wild-type IgG1 Fc crystal structures (e.g., 1HZH) to investigate the reasons for the reduced thermostability of the KIH model. It was found that KIH mutations decrease the number and strength of residue-residue contacts between the two CH3 domains.

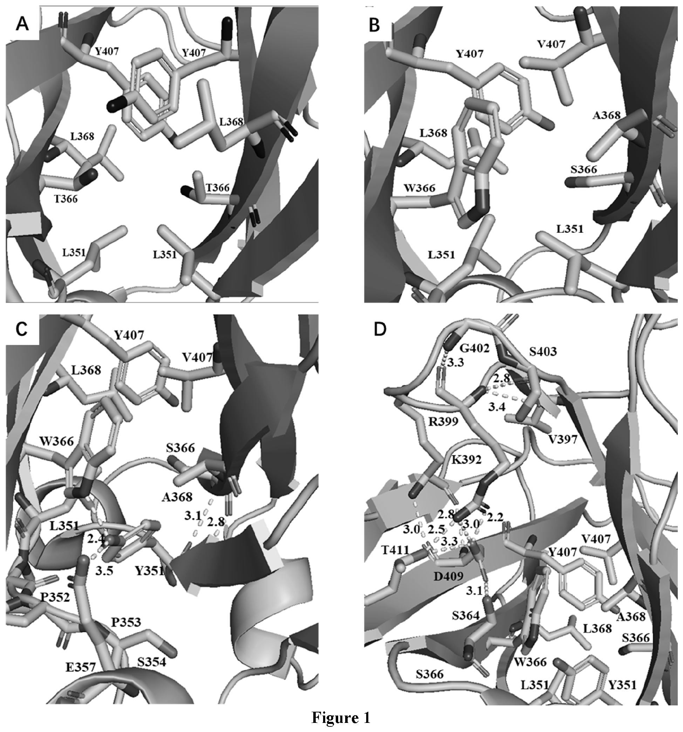

In wild-type IgG1 Fc, several key amino acids at the CH3-CH3 domain interface, including L351, L368, Y407, and others, form a hydrophobic core that covers a large surface area (see Figure 1A). In the KIH model, however, the surface area of the hydrophobic core is substantially reduced due to the L368A and Y407V mutations, which introduce amino acids with smaller side chains (see Figure 1B). Moreover, some residue-residue contacts surrounding the hydrophobic core are also perturbed. For example, in wild-type IgG1 Fc, the K409 side chain from one CH3 domain makes direct contacts with L368, K370, D399, F405, and Y407 in the other CH3 domain; in particular, K409 forms a complex hydrogen-bond network with D399, S364, T411, and a water molecule, which simultaneously prevents an electrostatic clash between K409 and K370. In the KIH structure, however, the K409 side chain in the knob chain is affected by the T366W mutation, resulting in loss of direct contact with L368A and Y407V, as well as loss of the stabilized water molecule and its mediated hydrogen-bond network.

To enhance CH3 interaction strength and improve thermal stability, amino acids adjacent to the Fc contact interface in the KIH model were preferentially selected as mutation targets, finally leading to the design of a series of Fc heterodimer mutation combinations. Specific amino acid mutation information is provided in Table 1. The KIH combination corresponds to the knobs-in-holes mutation combination disclosed in Patent US7951917B1, while M1-M7 represent newly designed candidate mutation combinations.

**Table 1. Design of Fc heterodimer mutation combinations, wherein Chain A is the chain comprising the knob mutation and Chain B is the chain comprising the corresponding hole mutation.**

| Combination | Chain A Name | Amino Acid Mutations of Chain A | SEQ ID NO. | Chain B Name | Amino Acid Mutations of Chain B | SEQ ID NO. |
|---|---|---|---|---|---|---|
| KIH | K0 | T366W | 1 | H0 | T366S-L368A-Y407V | 5 |
| M1 | K2 | T366W-K409D | 2 | H2 | T366S-L368A-Y407V-D399R | 6 |
| M2 | K0 | T366W | 1 | H8 | T366S-L368A-Y407V-L351Y | 7 |
| M3 | K2 | T366W-K409D | 2 | H7 | T366S-L368A-Y407V-L351Y-D399R | 8 |
| M4 | K9 | T366W-L351Y | 3 | H0 | T366S-L368A-Y407V | 5 |
| M5 | K7 | T366W-L351Y-K409D | 4 | H2 | T366S-L368A-Y407V-D399R | 6 |
| M6 | K2 | T366W-K409D | 2 | H2-D399K | T366S-L368A-Y407V-D399K | 9 |
| M7 | K2 | T366W-K409D | 2 | H7-D399K | T366S-L368A-Y407V-L351Y-D399K | 10 |

### Example 2: Expression and purification of VHH-Fc/Fc heterodimer

In this Example, to verify the effect of the Fc mutant combinations listed in Table 1 on promoting heterodimer formation, a Chain A VHH-Fc fusion construct and a Chain B Fc construct were designed. The VHH-Fc and Fc genes were synthesized and expression plasmids were constructed accordingly. After transfection of 293F cells, culture supernatants were harvested, and were purified by Protein A affinity chromatography. The purified proteins were analyzed by SDS-PAGE and SEC-HPLC to determine the heterodimer level and levels of other impurities, thereby assessing the effects of different mutation combinations on the expression yield and purity of the VHH-Fc/Fc heterodimer.

### Expression vector construction

Based on the amino acid sequence of the constant region of human immunoglobulin IgG1 (P01857) published in Uniprot protein database, the amino acid sequence of human IgG1-Fc was derived, comprising the hinge region, CH2 domain, and CH3 domain. A total of ten Fc mutant amino acid sequences were then designed according to Table 1 (see SEQ ID NO: 1 to SEQ ID NO: 10). A VHH fragment (derived from the Ozoralizumab antibody, see SEQ ID NO: 11) and a flexible linker polypeptide (see SEQ ID NO: 12) were introduced into the N-terminus of the Chain A Fc fragment to generate a Chain A VHH-Fc fusion molecule, while the Chain B Fc fragment was left unmodified. The difference in molecular weight between VHH-Fc and Fc facilitates distinguishing the proportions of AB heterodimers, AA/BB homodimers, A/B monomers, and high molecular weight species (HMWS) formed during recombinant expression.

The DNA-encoding genes for Chain A VHH-Fc and Chain B Fc were designed using codons optimized for mammalian/human expression, then cloned into the expression vector pcDNA3.4 (Invitrogen), yielding plasmids for the expression of VHH-Fc and Fc. Gene synthesis and plasmid construction were performed by Genwiz Suzhou Co., Ltd. (Genwiz).

### 293F Cell Transfection

VHH-Fc and Fc plasmids were mixed at a molar ratio of 1:1 or 0.6:1, followed by the addition of OPM-CD Trans293 medium (OPM, P82019) at a volume equivalent to 1/10 of the transfection volume. FectoPRO transfection reagent (PolyPlus, PT-116-010) was added to the culture medium at a ratio of 1 µl per 1.2 ml of cells. The medium containing the plasmids and the medium containing the transfection reagent were mixed and allowed to stand for 10 minutes. This mixture was then added to a shake flask containing Expi293F cells (Invitrogen, A14635), followed by shaking culture at 37°C under 8% CO₂. Sixteen hours post-transfection, a feed (OPM, F081918-001) was added at a volume equivalent to 10% of the transfection volume, and the culture was continued for an additional 6 days.

### Protein A Purification

A 1 ml Protein A Diamond chromatography column (Bestchrom, AA0273) was equilibrated with 5 column volumes of equilibration solution (20 mM sodium phosphate, pH 7.4). The clarified culture liquid was loaded onto the chromatography column at a flow rate of 0.5 column volumes per minute. Then the column packing was washed sequentially with 5 column volumes of equilibration solution (20 mM sodium phosphate, pH 7.4) and pre-elution solution (20 mM acetic acid-sodium acetate, pH 5.2). Finally, elution was performed with 10 column volumes of elution solution (20 mM acetic acid-sodium acetate, pH 3.2), and the collected eluate was adjusted to pH 6.0 using 1.5 M Tris base.

### SDS-PAGE Analysis

5µg of the Protein A column-purified product was taken and mixed with non-reducing loading buffer (without DTT), followed by electrophoresis on a 10% SurePAGE gel (Genscript, Cat. No. M00666). After electrophoresis, gels were stained with Coomassie Brilliant Blue, then destained and photographed using a gel imager.

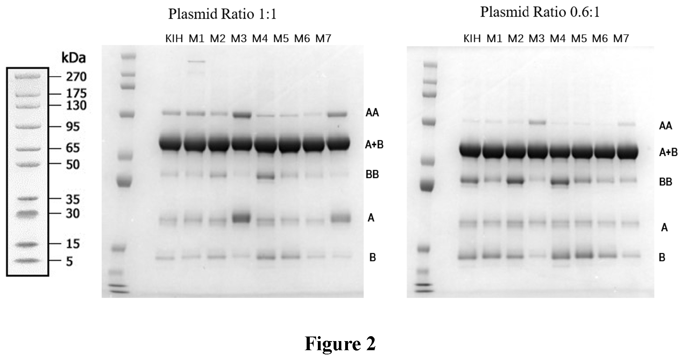

The results are shown in Figure 2. The co-expression products of the VHH-Fc and Fc plasmids contained predominantly five species with different molecular weights: AB heterodimers, AA homodimers, BB homodimers, A monomers, and B monomers. At a plasmid ratio of 1:1, the heterodimer content was high (>90%) for the KIH control and for combinations M1, M2, M5, and M6, whereas combinations M3, M4, and M7 showed lower heterodimer levels (80-90%). At a plasmid ratio of 0.6:1, the heterodimer content was high (>90%) for combinations M1, M3, M5, M6, and M7, while the KIH control and combinations M2 and M4 showed lower heterodimer levels (80-90%). These results indicate that, compared with the KIH control, combinations M1 and M6 more effectively promote AB heterodimer formation while suppressing AA and BB homodimer formation. Combination M2 shows a similar ability to the KIH control in promoting the formation of AB heterodimers as well as AA and BB homodimers. Combination M3 exhibits a comparable ability to promote AB heterodimer formation relative to the KIH control, but induces higher AA homodimer formation and lower BB homodimer formation.

### SEC-HPLC Analysis

SEC assays were performed on 1 mg/ml of the Protein A column-purified product using a Thermo Vanquish Core high-performance liquid chromatograph. The chromatographic column was a Waters BioResolve SEC mAb column (2.5 µm, 7.8 × 300 mm). PBS was used as the mobile phase for isocratic elution, with the following: elution time of 30 minutes, flow rate of 0.5 ml/min, detection wavelength of 280 nm, column temperature of 20°C, injection volume of 10 µl, and the temperature of the temperature-controlled tray set at 5 °C.

The results are shown in Table 2. Overall, the dimer proportions determined by SEC-HPLC for each mutant combination were consistent with the SDS-PAGE results. At a plasmid ratio of 1:1, the heterodimer content was high (>90%) for the KIH control and combinations M1, M2, M5, and M6, whereas combinations M3, M4, and M7 showed lower heterodimer levels (80-90%). At a plasmid ratio of 0.6:1, the heterodimer content was high (>90%) for combinations M1, M3, M5, M6, and M7, while the KIH control and combinations M2 and M4 showed lower heterodimer levels (80-90%).

**Table 2: SEC-HPLC analysis of VHH-Fc/Fc mutant combinations**

| Combination | Plasmid Ratio | Expression Level (mg/ml) | SEC Purity (%) | Plasmid Ratio | Expression Level (mg/ml) | SEC Purity (%) |
|---|---|---|---|---|---|---|
| KIH | 1: 1 | 590 | 94.16 | 0.6 : 1 | 645 | 85.86 |
| M1 | 1: 1 | 815 | 94.51 | 0.6 : 1 | 575 | 93.30 |
| M2 | 1: 1 | 670 | 94.75 | 0.6 : 1 | 630 | 85.86 |
| M3 | 1: 1 | 585 | 80.90 | 0.6 : 1 | 575 | 93.99 |
| M4 | 1: 1 | 635 | 89.95 | 0.6 : 1 | 580 | 83.63 |
| M5 | 1: 1 | 565 | 95.33 | 0.6 : 1 | 575 | 90.26 |
| M6 | 1: 1 | 477 | 98.09 | 0.6 : 1 | 351 | 93.78 |
| M7 | 1: 1 | 409 | 86.87 | 0.6 : 1 | 548 | 96.12 |

Taken together, these results indicate that introducing the Hole L351Y mutation into the KIH background does not substantially change the proportions of heterodimers and homodimers. Moreover, introducing the K409D/D399R double mutation into the KIH-L351Y background does not substantially change the heterodimer proportion; however, it leads to an increased proportion of AA homodimers and a decreased proportion of BB homodimers.

### Example 3: Thermal stability analysis of VHH-Fc/Fc heterodimer

### Heterodimer Purification

A 1 mL Mono S ion exchange column (Cytiva, 17516801) was washed with equilibration solution (20 mM sodium phosphate, pH 6.0) for 5 column volumes or until the baseline was achieved. Protein A affinity-purified samples were adjusted to pH 6.0 using either 1.5 M Tris base or 50 mM sodium acetate (pH 3.2) and loaded onto the Mono S column at a flow rate of 0.5 column volumes per minute. The column was then washed with ten column volumes of equilibration solution. Finally, elution was performed using elution buffer (20 mM sodium phosphate + 1 M sodium chloride, pH 6.0) in a 0-30% gradient, and eluate fractions were collected. The purity of the heterodimer in each fraction was analyzed by SEC-HPLC, and fractions with a purity greater than 98% were pooled.

### Thermal Stability Detection

Thermal stability of the samples was assessed using a micro-differential scanning calorimeter (Microcal PEAQ-DSC) as follows. Protein samples were buffer-exchanged into PBS and diluted to 1 mg/mL. The detection temperature range was set from 20 °C to 100 °C at a ramp rate of 90 °C/h. PBS was used as a blank buffer and loaded into the measurement cell for temperature scanning. The solution in the sample cell was then removed, the test sample was added for temperature scanning. Experimental data were processed using the instrument's built-in analysis software to determine the Tm value.

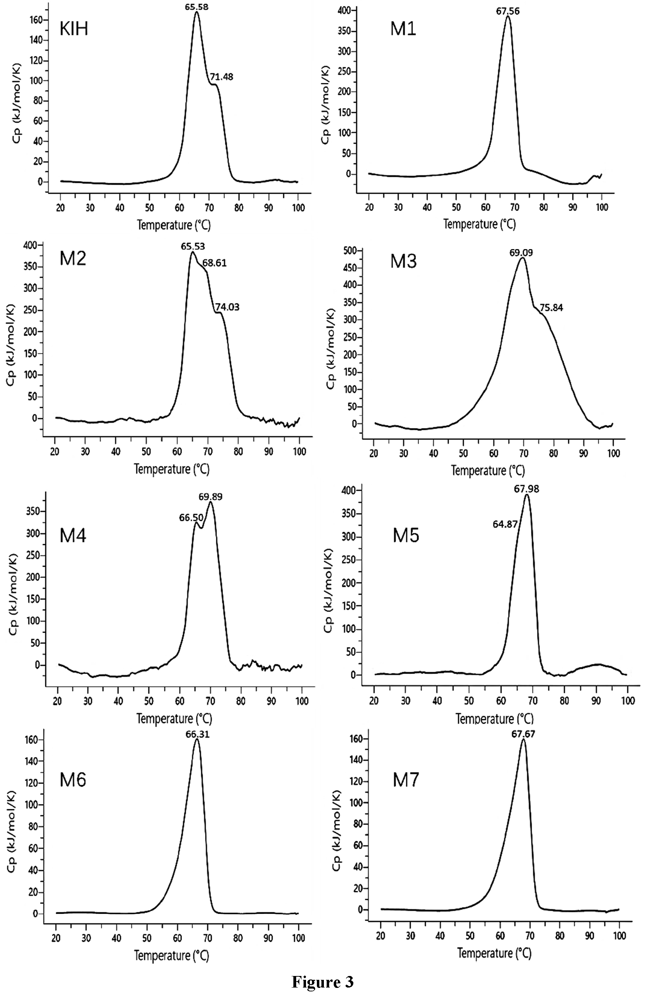

The Tm determination results for the VHH-Fc/Fc mutant combinations are shown in Table 3 and Figure 3. The KIH control exhibited at least two Tm values: the first Tm (Tm1) of 65.68 °C, reflecting the thermal stability of the VHH fragment and the CH2 domain, and the second Tm (Tm2) of 71.48 °C, reflecting the thermal stability of the CH3 domain. The Tm values of mutant combinations M1 and M6 were 67.56 °C and 66.31 °C, respectively, both markedly lower than that of the KIH control. These results suggest that introducing the electrostatic steering design into the KIH structure unexpectedly reduced CH3-domain thermal stability. Interestingly, the CH3-domain Tm of mutant combination M2 was about 74.03 °C, representing an increase of about 2.5 °C relative to the KIH control; and the CH3-domain Tm of mutant combination M3 was even higher, reaching about 75.84 °C. These results indicate that the single Fc L351Y mutation in chain B improves CH3-domain stability in the KIH format. Furthermore, combining this mutation with Fc K409D in chain A and Fc D399R in chain B produced a synergistic effect, further enhancing CH3-domain stability. Other mutant combinations, including M4, M5, and M7, showed poor thermostability, with Tm values all below 70 °C. These findings indicate that, in the KIH background, incorporation of L351Y in chain A or L351Y-D399K in chain B failed to improve CH3-domain thermostability.

**Table 3. Tm values of VHH-Fc/Fc mutant combinations**

| Combination | Tm1 Value | Tm2 Value |
|---|---|---|
| KIH | 65.58 | 71.48 |
| M1 | / | 67.56 |
| M2 | 65.53 | 74.03 |
| M3 | 69.09 | 75.84 |
| M4 | 66.50 | 69.89 |
| M5 | 64.87 | 67.98 |
| M6 | / | 66.31 |
| M7 | / | 67.67 |

### Example 4: Expression and purification of VHH-Fc/Fab-Fc heterodimers

To assess the general applicability of the mutant combinations described in Example 2 across different antibody sequences and molecular formats, a VHH-Fc/Fab-Fc heterodimer was constructed. A VHH fragment (amino acid sequence shown in SEQ ID NO: 11) and a flexible linker (SEQ ID NO: 12) were fused to the N-terminus of the first Fc fragment to generate an A-chain VHH-Fc fusion protein. A Fab fragment was fused to the N-terminus of the second Fc fragment to generate an Fab-Fc half-antibody. The Fab sequence was derived from a B7-H3 mouse monoclonal antibody obtained by hybridoma technology and comprises a light-chain domain (VL-CL; amino acid sequence shown in SEQ ID NO: 14) and a heavy-chain domain (VH-CH1; amino acid sequence shown in SEQ ID NO: 13). In the Fab-Fc molecule, the heavy chain was designated as chain B and the light chain as chain C. The molecular-weight difference between the VHH-Fc fusion protein and the Fab-Fc half-antibody facilitates discrimination of the different species formed during recombinant expression, including ABC heterodimers, AA homodimers, BB homodimers, BBCC homodimers, A monomers, B monomers, and C monomers. The ABC heterodimer was the desired bispecific antibody, and the other species were impurities.

Similar to the procedure described in Example 2, genes encoding chains A, B, and C were synthesized, and expression plasmids for each chain were constructed. 293F cells were transfected, and the culture supernatant was harvested and purified by Protein A affinity chromatography. The purified products were analyzed by SDS-PAGE and SEC-HPLC to determine the content of the ABC heterodimer and other impurity species.

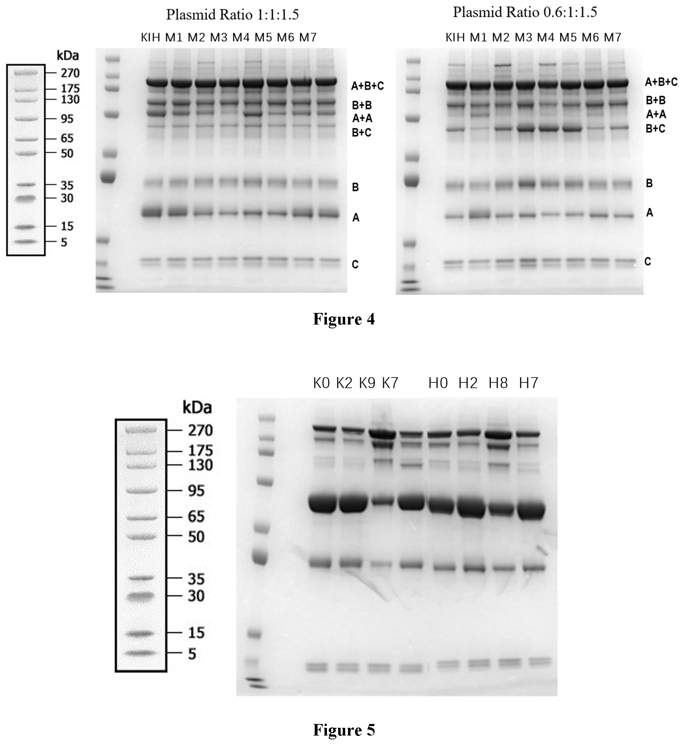

The SDS-PAGE results are shown in Figure 4. Two plasmid transfection ratios (A-chain plasmid : B-chain plasmid : C-chain plasmid) were used: 1:1:1.5 and 0.6:1:1.5. Under both conditions, the ABC heterodimer purity of the KIH control was below 90%. In contrast, mutant combinations M1, M2, M3, M5, M6, and M7 achieved ABC heterodimer purity above 90% under at least one condition. However, mutant combination M4 performed poorly, with ABC heterodimer purity lower than that of the KIH control under both conditions.

The SEC-HPLC results are shown in Table 4. At both A:B:C plasmid ratios of 1:1:1.5 and 0.6:1:1.5, all M1-M7 mutant combinations yielded expression levels above 200 mg/L, comparable to or higher than that of the KIH control. The ABC heterodimer proportions determined by SEC-HPLC for each mutant combination were generally consistent with the SDS-PAGE results. Under both conditions, the ABC heterodimer purity of the KIH control was below 90%. At the A:B:C plasmid ratio of 1:1:1.5, all M1-M7 mutant combinations showed higher ABC heterodimer proportions than the KIH control, with M1, M2, M3, M5, and M7 exceeding 90%. At the A:B:C plasmid ratio of 0.6:1:1.5, M1, M6, and M7 exhibited higher heterodimer proportions than the KIH control, and each exceeded 90%.

**Table 4. SEC-HPLC analysis of VHH-Fc/Fab-Fc mutant combinations**

| Combination | plasmid ratio | expression level (mg/L) | SEC purity | plasmid ratio | expression level (mg/L) | SEC purity |
|---|---|---|---|---|---|---|
| KIH | 1:1:1.5 | 300 | 80.51 | 0.6:1:1.5 | 258 | 87.61 |
| M1 | 1:1:1.5 | 400 | 90.44 | 0.6:1:1.5 | 500 | 93.71 |
| M2 | 1:1:1.5 | 420 | 90.70 | 0.6:1:1.5 | 350 | 81.07 |
| M3 | 1:1:1.5 | 500 | 94.97 | 0.6:1:1.5 | 285 | 75.86 |
| M4 | 1:1:1.5 | 400 | 86.15 | 0.6:1:1.5 | 301 | 69.46 |
| M5 | 1:1:1.5 | 350 | 91.81 | 0.6:1:1.5 | 302 | 79.7 |
| M6 | 1:1:1.5 | 259 | 86.54 | 0.6:1:1.5 | 213 | 95.36 |
| M7 | 1:1:1.5 | 266 | 91.1 | 0.6:1:1.5 | 283 | 94.78 |

### Example 5: Expression and purification of Fab-Fc half-antibody

To assess the general applicability of the mutant combinations described in Example 2 across different antibody sequences and molecular formats, two Fab-Fc half-antibody mutants were constructed by fusing two Fab fragments that bind distinct antigens to the Fc regions. The A-chain Fc designed in Table 1 of Example 1 was fused at its N-terminus to the first Fab to generate the A half-antibody, wherein the Fab was derived from the anti-EGFR monoclonal antibody zalutumumab and comprises a heavy-chain domain (VH-CH1, amino acid sequence shown in SEQ ID NO: 15) and a light-chain domain (VL-CL, amino acid sequence shown in SEQ ID NO: 16). The B-chain Fc designed in Table 1 of Example 1 was fused at its N-terminus to the second Fab to generate the B half-antibody, wherein the Fab was derived from the anti-HER2 monoclonal antibody pertuzumab and comprises a heavy-chain domain (VH-CH1, amino acid sequence shown in SEQ ID NO: 17) and a light-chain domain (VL-CL, amino acid sequence shown in SEQ ID NO: 18).

Similar to the procedure described in Example 2, genes encoding the light chains and heavy chains of A and B half-antibodies were synthesized. Plasmids expressing the respective genes were constructed and transfected into 293F cells for expression of the A and B half-antibodies, respectively. The culture supernatant was harvested and subjected to Protein A affinity purification. The purified products were analyzed by SDS-PAGE and SEC-HPLC.

The SDS-PAGE analysis results are shown in Figure 5. For all tested half-antibody mutants, the major expression products in 293 cells were half-antibody monomers with a molecular weight of 75 kDa and homodimers with a molecular weight of 150 kDa, together with minor amounts of light chains with a molecular weight of 25 kDa and heavy chains with a molecular weight of 50 kDa. Among these, the Fab-Fc mutants K0, K2, K7, H0, H2, and H7 exhibited a monomer proportion greater than 80%, whereas mutants K9 and H8 showed a monomer proportion below 50%.

The SEC-HPLC analysis results are shown in Table 5. All half-antibody mutants were expressed at levels greater than 100 mg/L in 293 cells. The proportions of half-antibody monomers, homodimers, and aggregates in the expression products of 293 cells were calculated using the built-in SEC-HPLC analysis software. For all Fab-Fc mutants, the combined content of the produced monomer and dimer exceeded 95%, while the content of aggregates was less than 5%. K0 yielded 46.27% dimers and K2 yielded 56.17% dimers, which should include both non-covalent dimers and disulfide-linked covalent dimers. These results suggest that the K409D mutation exerted virtually no effect on CH3-domain homodimerization. K9 produced about 86.2% dimers, which was significantly higher than that of K0, indicating that the L351Y mutation promoted CH3-domain homodimerization. K7 yielded 26.51% dimers and 73.24% monomers. H0 yielded 30.4% dimers, which should include both non-covalent and disulfide-linked covalent dimers. H2 yielded 14.38% dimers and H7 yielded 15.05% dimers. H2 and H7 produced significantly fewer homodimers than H0, indicating that the D399R mutation prevented CH3-domain homodimerization via electrostatic repulsion. H8 yielded 64.71% dimers, which was significantly higher than that of H0, indicating that the L351Y mutation promoted CH3-domain homodimerization.

**Table 5. SEC-HPLC analysis results of Fab-Fc half-antibodies**

| Half-antibody name | Expression level (mg/L) | Monomer proportion | Dimer proportion | Aggregate proportion |
|---|---|---|---|---|
| K0 | 290 | 52.22 | 46.27 | 1.51 |
| K2 | 260 | 39.46 | 56.17 | 4.36 |
| K9 | 260 | 13.32 | 86.20 | 0.48 |
| K7 | 120 | 73.24 | 26.51 | 0.25 |
| H0 | 290 | 68.93 | 30.40 | 0.68 |
| H2 | 170 | 85.62 | 14.38 | 0 |
| H8 | 300 | 35.18 | 64.71 | 0.12 |
| H7 | 120 | 84.92 | 15.08 | 0 |

### Example 6: Preparation and purification of Fab-Fc/Fab-Fc heterodimers

### In vitro assembly

The half-antibody solutions obtained by affinity chromatography in Example 5 were mixed at an equimolar ratio (1:1) according to the respective half-antibody combinations shown in Table 6, and then neutralized to pH 8-8.2 with 1.5 M Tris base. The total protein content of the reaction system was 2 mg. After standing at room temperature for 30 minutes, 200x molar ratio GSH solution (Sigma, S0073) was added. The reaction samples were incubated statically at 37 °C for 4 hours, and aliquots were collected at 0.5, 1, 2, and 4 hours to immediately determine the *in vitro* assembly efficiency. The reaction samples were then transferred to a 4 °C refrigerator and incubated overnight.

### Purity Analysis by HIC-HPLC (Hydrophobic Interaction High-Performance Liquid Chromatography)

HIC was performed on a Thermo Vanquish Core HPLC system using a TOSOH TSKgel Butyl-NPR chromatographic column (2.5 µm, 4.6 × 100 mm). Gradient elution was carried out with mobile phase A (a mixture of 1.5 M ammonium sulfate and 20 mM phosphate, pH 7.0) and mobile phase B (20 mM phosphate buffer, pH 7.0) with the following: 0-15min 0% B to 100% B, 15.5-22min 0% B to 0% B. The flow rate was 0.6 mL/min, the detection wavelength was 280 nm, the column temperature was 25 °C, the injection volume was 10 µL, and the temperature-controlled tray was set to 5 °C.

The HIC-HPLC chromatograms of the half-antibodies and the bispecific antibodies collected after 4 hours of reaction are shown in Figure 6. This method enables sensitive assessment of the assembly efficiency of bispecific antibody heterodimer molecules. Monomers and homodimers derived from the half-antibodies differ in hydrophobicity and therefore exhibited distinct retention times in the HIC chromatograms. Bispecific antibodies assembled in vitro from the two Fab-Fc half-antibodies also showed retention times distinct from those of the half-antibodies.

The HIC-HPLC results are shown in Table 6. After incubation at 37 °C for 0.5 h, the proportion of AB heterodimers formed by the KIH control and the M1, M2, and M3 mutant combinations exceeded 90%. After incubation at 37 °C for 2 h and 4 h, the AB heterodimer proportions for KIH, M1, M2, and M3 increased to about 95%.

**Table 6. HIC-HPLC analysis of assembly efficiency of bispecific antibodies**

| | 37°C incubation time | | | |
|---|---|---|---|---|
| Combination | 0.5 hour | 1 hour | 2 hours | 4 hours |
| KIH | 91.47 | 92.37 | 95.44 | 95.69 |
| M1 | 92.28 | 93.63 | 93.65 | 93.72 |
| M2 | 91.79 | 92.66 | 93.39 | 94.74 |
| M3 | 93.95 | 94.27 | 95.51 | 96.83 |

### Heterodimer Purification

A 1 mL Capto S ImpAct ion exchange column (Cytiva, 17371751) was washed with equilibration solution (20 mM sodium phosphate, pH 6.0) for 5 column volumes or until the baseline was achieved. The in vitro assembled samples were adjusted to pH 5.8-6.2 with 1 M acetic acid, diluted 1-2 fold with ultrapure water, and filtered through a 0.22 µm PES filter membrane. The samples were loaded onto the Capto S column at a flow rate of 0.5 column volumes per minute, followed by washing with 10 CV of the equilibration solution. Finally, elution was performed using elution buffer (20 mM sodium phosphate + 1 M sodium chloride, pH 6.0) in a 0-30% gradient, and eluate fractions were collected. The purity of the dimer peak in each fraction was analyzed by SEC-HPLC, and fractions with a purity greater than 98% were pooled.

The SEC-HPLC results are shown in Table 7. Under incubation conditions of 37°C for 4 hours, the proportion of dimers produced by the KIH, and the M1, M2, and M3 mutant combinations exceeded 96%, with AB heterodimers as the major component and a small amount of AA or BB homodimers. Other impurities included A or B monomers, or high molecular weight aggregates, all accounting for less than 2%.

**Table 7. Purity analysis of bispecific antibodies by SEC-HPLC after incubation at 37 °C for 4 h**

| Combinations | Aggregate | Dimer | Monomer |
|---|---|---|---|
| KIH | 1.05 | 98.09 | 0.86 |
| M1 | 0.73 | 97.99 | 1.28 |
| M2 | 1.28 | 96.84 | 1.88 |
| M3 | 1.28 | 96.89 | 1.83 |

### Example 7. Antigen Binding Activity of Bispecific Antibody

The antigen-binding activity of the purified bispecific antibodies from Example 6 was examined. Recombinant human EGFR protein (ACRO Biosystems, EGR-H5222) and HER2 protein (ACRO Biosystems, HE2-H5225) were diluted to 1 µg/mL with coating buffer (Solarbio, C1055), and 100 µL was added to each well. The plates were incubated at 4°C for 16 hours. Blocking agent was prepared by supplementing PBS with 3% BSA (Sangon Biotech, 9048-46-8) and 0.05% Tween 20 (Sangon Biotech, A600560-0500). After washing the plates three times with 0.05% PBST, 300 µL of blocking buffer was added to each well, followed by incubation at room temperature for 2 hours. The bispecific antibodies were serially diluted 3-fold in 0.5% BSA-0.05% PBST blocking agent, starting at a maximum concentration of 15 µg/mL. After washing the plates three times with 0.05% PBST, 100 µL of the bispecific antibody solution was added to each well and incubated at room temperature for 1 hour. After washing three times with 0.05% PBST, 100 µL of HRP-labeled secondary antibody (Bethyl, A80-304P, 1:5000 dilution) was added to each well and incubated at room temperature for 1 hour. After three washes with 0.05% PBST, 100 µL of chromogenic reagent (Solarbio, PR1210) was added to each well, and incubated at room temperature in the dark for 10 min. After adding 100 µL of stop solution (Solarbio, C1058) to each well, the absorbance at OD450 was measured using a multimode microplate reader (Tecan, Spark).

The ELISA results are shown in Figure 7. The bispecific antibodies generated from the KIH, and from the M1, M2, and M3 mutant combinations exhibited highly similar antigen-binding curves. The IC50 values of these bispecific antibodies for EGFR binding ranged from 17.0 to 24.9 ng/mL, and those for HER2 binding ranged from 12.8 to 14.6 ng/mL. These results indicate that the mutations in the CH3 domain of Fc region do not affect the antigen-binding ability of the bispecific antibodies.

### Example 8. Binding Activity of Bispecific Antibodies to Fc Receptors

The binding affinities of the purified bispecific antibodies from Example 6 to human FcγRI receptor and human FcRn receptor were determined by bio-layer interferometry (BLI). Detection was performed using a Fortebio Octet Red 96 instrument, and the equilibrium dissociation constant (KD) was calculated.

One column of HIS1K sensors (Fortebio, Cat. 18-5120) were immersed in standard buffer (1×PBS, pH 7.4 with 0.1% BSA and 0.02% Tween-20). After equilibration of the biosensors in the standard buffer, 5 µg/mL recombinant human FcγRI protein (ACRO Biosystems, Cat. FCA-H52H1) was immobilized on the biosensors, followed by association with 100 nM antibody and dissociation in the standard buffer. The instrument operation steps were as follows: Baseline 1 (60 s), Loading (~15 s, 0.5 nm), Baseline 2 (60 s), Association (60 s), and Dissociation (120 s), with a rotation speed of 1000 rpm and a temperature of 30 °C.

One column of SA sensors (Fortebio, Cat 18-5019) were immersed in pH 6.0 buffer (1×PBS, pH 6.0, with 0.1% BSA, 0.02% Tween-20). After equilibration of the biosensors in pH 6.0 buffer, 200 µL of pH 6.0 buffer, 100 nM antibody, and human FcRn were dispensed separately into the wells of a black 96-well polystyrene microplate (Greiner, 655209). Detection was performed on a Fortebio Octet Red96. The plate layout was set up according to the sample positions, and the sensor positions were selected. After equilibration of the biosensors in pH 6.0 buffer, 2 µg/mL recombinant human FcRn (ACROBiosystems Cat FCM-H8286) was immobilized on the biosensors, followed by association with 100 nM antibody and dissociation in pH 6.0 buffer. The instrument operation steps were as follows: Baseline1 (60 s), Loading (~100 s, 3.0 nm), Baseline2 (60 s), Association (60 s) and Dissociation (60 s), with a rotation speed of 1000 rpm and a temperature of 30°C.

The Fortebio assay results are shown in Figure 8. The affinity of the control antibody IgG1 for human FcγRI was 6.33 × 10⁻⁹ M; and the affinities of the KIH, and of the M1, M2, and M3 mutant combinations for human FcγRI ranged from 1.01 × 10⁻⁸ M to 1.03 × 10⁻⁸ M. The affinity of the control antibody IgG1 for human FcRn was 1.41 × 10⁻⁸ M; the affinities of the KIH, M1, M2, and M3 mutant combinations for FcRn were slightly lower, ranging from 1.44 × 10⁻⁸ M to 1.85 × 10⁻⁸ M. These differences in affinity were within the instrument's measurement error. Therefore, the Fc mutant combinations obtained in this study basically maintained binding to human FcRn and human FcγRI. Furthermore, the affinity values of the M1, M2, and M3 mutant combinations were highly similar to those of the KIH control.

### Example 9: Accelerated Stability Test of Bispecific Antibody

The purified bispecific antibodies from Example 6 were buffer-exchanged into 20 mM histidine and diluted to 1 mg/ml, then filtered through a 0.22 µm PES membrane. Samples were placed in a 42°C oven, and samples were taken on days 7 and 14 for purity analysis by SEC-HPLC and SDS-PAGE.

The SEC-HPLC results are shown in Table 9. After storage at 42 °C for 7 and 14 days, the heterodimer purity of each mutant combination decreased only slightly compared with that at day 0. Specifically, for the KIH, M1, M2, and M3 mutant combinations, the purities decreased by 0.21%, 0.73%, 0.47%, and 0.57% on day 7, respectively, and by 1.43%, 1.50%, 1.46%, and 0.84% on day 14, respectively. These results indicate that the newly designed Fc mutant combinations exhibited superior stability under high-temperature storage conditions.

SDS-PAGE results also demonstrated that the proportions of protein bands at different molecular weights remained essentially unchanged for the KIH, M1, M2, and M3 mutant combinations after storage at 42 °C for 14 days, compared with day 0.

**Table 9. Storage stability of bispecific antibodies in accelerated experiments as analyzed by SEC-HPLC**

| Mutant Combination | 0-day heterodimer | 7-day heterodimer | Change at day 7 | 14-day heterodimer | Change at day 14 |
|---|---|---|---|---|---|
| KIH | 99.13% | 98.92% | -0.21% | 97.70% | -1.43% |
| M1 | 99.15% | 98.43% | -0.73% | 97.65% | -1.50% |
| M2 | 98.89% | 98.42% | -0.47% | 97.43% | -1.46% |
| M3 | 99.66% | 99.09% | -0.57% | 98.82% | -0.84% |

### Discussion

By analyzing the reasons underlying the reduced thermal stability of the knobs-into-holes (KiH) model, we selected amino acids near the CH3 interface of Fc fragment as mutation sites to evaluate how different mutation combinations affect the manufacturability, physicochemical properties, and biological functions of Fc-based IgG-like mono- and multi-specific antibodies.

Without wishing to be bound by theory, it is believed that, in wild-type IgG1 Fc, residue L351 on one chain makes direct contacts with residues L351, P352, P353, S354, and T366 on the opposing chain. In the knobs-into-holes (KIH) structure, L351 on the hole chain makes direct contacts with residues L351, P352, P353, S354, and T366W on the knob chain; however, the contact distance is increased, and the interaction is expected to be weakened. Mutation of hole-chain L351, for example to tyrosine with a larger side chain (L351Y), is hypothesized to tighten contacts between hole-chain residue 351 and knob-chain residues P352, P353, S354, and T366W, thereby enhancing hydrophobic interactions (see Figure 1C); and to create new contacts with other knob-chain residues, for example, the hydroxyl (OH) group of L351Y forms a new hydrogen-bonding network with T366W and E357.

On the basis of the hole-chain L351 mutation, a combination of knob-chain K409D and hole-chain D399R mutations was further introduced. Without wishing to be bound by theory, it is hypothesized that this mutation combination may give rise to a salt-bridge pair similar to that in an IgG1 Fc; and that the hole-chain D399R forms new contacts with knob-chain residues K392, T411, and T366W, whereas the knob-chain K409D mutation remains direct contact with hole-chain F405 (see Figure 1D).

The experimental results in the above Examples demonstrate that, in the KIH structure, incorporation of the above mutations-namely, hole-chain L351Y, or hole-chain L351Y + knob-chain K409D and hole-chain D399R-provides beneficial effects on the manufacturability and physicochemical properties of heterodimeric Fc scaffolds and monospecific/multi-specific proteins (particularly bispecific antibodies) comprising the same. Following cellular expression and affinity chromatography purification of the Fc heterodimeric scaffold proteins comprising said mutation combinations, the Fc heterodimer proportion can exceed 90%, which is higher than that of a control Fc protein comprising only the corresponding knobs-into-holes mutations; and, after further purification by ion-exchange chromatography, the purity can exceed 98%. Moreover, compared with the control Fc protein, the purified Fc heterodimeric scaffold proteins exhibit improved thermal stability, with the CH3-domain Tm determined by DSC being at least 74 °C.

Furthermore, the experimental results of the above Examples also demonstrate that, in the KIH structure, incorporation of the above mutations-namely, hole-chain L351Y, or hole-chain L351Y + knob-chain K409D and hole-chain D399R-has essentially no effect on antigen-binding activity of the heterodimeric Fc scaffold or mono-multi-specific proteins (particularly bispecific antibodies) comprising the same, nor on binding to FcRn and FcγR.

Taken together, these results indicate that, in a KIH background, introduction of the Hole L351Y mutation, or the combined mutations of Hole L351Y-D399R with Knob K409D, is beneficial for improving manufacturability and stability of a desired heterodimeric antibody.

### Sequence Listing

| SEQ ID NO: | Description/Name of Sequence | Amino acid/nucleotide sequence |
|---|---|---|
| 1 | Fc K0 mutation (knob) (T366W) (EU 221-447) | DKTHTCPPCPAPELLGGPSVFLFPPKPKDTLMISRTPEVTCVVVDVS HEDPEVKFNWYVDGVEVHNAKTKPREEQYNSTYRVVSVLTVLHQ DWLNGKEYKCKVSNKALPAPIEKTISKAKGQPREPQVYTLPPSREE MTKNQVSLWCLVKGFYPSDIAVEWESNGQPENNYKTTPPVLDSDG SFFLYSKLTVDKSRWQQGNVFSCSVMHEALHNHYTQKSLSLSPGK |
| 2 | Fc K2 mutation (K409D) | DKTHTCPPCPAPELLGGPSVFLFPPKPKDTLMISRTPEVTCVVVDVS HEDPEVKFNWYVDGVEVHNAKTKPREEQYNSTYRVVSVLTVLHQ DWLNGKEYKCKVSNKALPAPIEKTISKAKGQPREPQVYTLPPSREE MTKNQVSLWCLVKGFYPSDIAVEWESNGQPENNYKTTPPVLDSDG SFFLYSDLTVDKSRWQQGNVFSCSVMHEALHNHYTQKSLSLSPGK |
| 3 | Fc K9 mutation (L351Y) | DKTHTCPPCPAPELLGGPSVFLFPPKPKDTLMISRTPEVTCVVVDVS HEDPEVKFNWYVDGVEVHNAKTKPREEQYNSTYRVVSVLTVLHQ DWLNGKEYKCKVSNKALPAPIEKTISKAKGQPREPQVYTYPPSREE MTKNQVSLWCLVKGFYPSDIAVEWESNGQPENNYKTTPPVLDSDG SFFLYSKLTVDKSRWQQGNVFSCSVMHEALHNHYTQKSLSLSPGK |
| 4 | Fc K7 mutation (L351Y-K409D) | DKTHTCPPCPAPELLGGPSVFLFPPKPKDTLMISRTPEVTCVVVDVS HEDPEVKFNWYVDGVEVHNAKTKPREEQYNSTYRVVSVLTVLHQ DWLNGKEYKCKVSNKALPAPIEKTISKAKGQPREPQVYTYPPSREE MTKNQVSLWCLVKGFYPSDIAVEWESNGQPENNYKTTPPVLDSDG SFFLYSDLTVDKSRWQQGNVFSCSVMHEALHNHYTQKSLSLSPGK |
| 5 | Fc H0 mutation (hole) (T366S-L368AY407V) | DKTHTCPPCPAPELLGGPSVFLFPPKPKDTLMISRTPEVTCVVVDVS HEDPEVKFNWYVDGVEVHNAKTKPREEQYNSTYRVVSVLTVLHQ DWLNGKEYKCKVSNKALPAPIEKTISKAKGQPREPQVYTLPPSREE MTKNQVSLSCAVKGFYPSDIAVEWESNGQPENNYKTTPPVLDSDG SFFLVSKLTVDKSRWQQGNVFSCSVMHEALHNHYTQKSLSLSPGK |
| 6 | Fc H2 mutation (D399R) | DKTHTCPPCPAPELLGGPSVFLFPPKPKDTLMISRTPEVTCVVVDVS HEDPEVKFNWYVDGVEVHNAKTKPREEQYNSTYRVVSVLTVLHQ DWLNGKEYKCKVSNKALPAPIEKTISKAKGQPREPQVYTLPPSREE MTKNQVSLSCAVKGFYPSDIAVEWESNGQPENNYKTTPPVLRSDG SFFLVSKLTVDKSRWQQGNVFSCSVMHEALHNHYTQKSLSLSPGK |
| 7 | Fc H8 mutation (L351Y) | DKTHTCPPCPAPELLGGPSVFLFPPKPKDTLMISRTPEVTCVVVDVS HEDPEVKFNWYVDGVEVHNAKTKPREEQYNSTYRVVSVLTVLHQ DWLNGKEYKCKVSNKALPAPIEKTISKAKGQPREPQVYTYPPSREE MTKNQVSLSCAVKGFYPSDIAVEWESNGQPENNYKTTPPVLDSDG SFFLVSKLTVDKSRWQQGNVFSCSVMHEALHNHYTQKSLSLSPGK |
| 8 | Fc H7 mutation (L351Y-D399R) | DKTHTCPPCPAPELLGGPSVFLFPPKPKDTLMISRTPEVTCVVVDVS HEDPEVKFNWYVDGVEVHNAKTKPREEQYNSTYRVVSVLTVLHQ DWLNGKEYKCKVSNKALPAPIEKTISKAKGQPREPQVYTYPPSREE MTKNQVSLSCAVKGFYPSDIAVEWESNGQPENNYKTTPPVLRSDG SFFLVSKLTVDKSRWQQGNVFSCSVMHEALHNHYTQKSLSLSPGK |
| 9 | Fc H2-D399K mutation | DKTHTCPPCPAPELLGGPSVFLFPPKPKDTLMISRTPEVTCVVVDVS |
| | | HEDPEVKFNWYVDGVEVHNAKTKPREEQYNSTYRVVSVLTVLHQ DWLNGKEYKCKVSNKALPAPIEKTISKAKGQPREPQVYTLPPSREE MTKNQVSLSCAVKGFYPSDIAVEWESNGQPENNYKTTPPVLKSDG SFFLVSKLTVDKSRWQQGNVFSCSVMHEALHNHYTQKSLSLSPGK |
| 10 | Fc H7-D399K mutation (L351Y-D399K) | DKTHTCPPCPAPELLGGPSVFLFPPKPKDTLMISRTPEVTCVVVDVS HEDPEVKFNWYVDGVEVHNAKTKPREEQYNSTYRVVSVLTVLHQ DWLNGKEYKCKVSNKALPAPIEKTISKAKGQPREPQVYTYPPSREE MTKNQVSLSCAVKGFYPSDIAVEWESNGQPENNYKTTPPVLKSDG SFFLVSKLTVDKSRWQQGNVFSCSVMHEALHNHYTQKSLSLSPGK |
| 11 | Ozoralizumab VHH | |
| 12 | Linker | GGGGSGGGGS |
| 13 | hz32F8.9- VH+CH1 | QVQLVQSGAEVKKPGSSVKVSCKTSGYTFTDYVITWVRQAPGQG LEWMGEIYPRSDRTYYNENFKGRVTITADKSTNTAYMELSSLRSED TAVYYCARWDNYYFDYWGQGTLVTVSSASTKGPSVFPLAPSSKST SGGTAALGCLVKDYFPEPVTVSWNSGALTSGVHTFPAVLQSSGLYS LSSVVTVPSSSLGTQTYICNVNHKPSNTKVDKKVEPKSC |
| 14 | hz32F8.9-VL+CL | DVVMTQSPLSLPVTLGQPASISCRSSQSLVHSNAITYLHWYQQRPG QSPRLLIYKVSNRFSGVPDRFSGSGSGTDFTLKISRVEAEDVGVYY CSQTTHVPWTFGGGTKVEIKRTVAAPSVFIFPPSDEQLKSGTASVVC LLNNFYPREAKVQWKVDNALQSGNSQESVTEQDSKDSTYSLSSTL TLSKADYEKHKVYACEVTHQGLSSPVTKSFNRGEC |
| 15 | Zalutumumab-VH+CH1 | QVQLVESGGGVVQPGRSLRLSCAASGFTFSTYGMHWVRQAPGKG LEWVAVIWDDGSYKYYGDSVKGRFTISRDNSKNTLYLQMNSLRAE DTAVYYCARDGITMVRGVMKDYFDYWGQGTLVTVSSASTKGPSV FPLAPSSKSTSGGTAALGCLVKDYFPEPVTVSWNSGALTSGVHTFP AVLQSSGLYSLSSVVTVPSSSLGTQTYICNVNHKPSNTKVDKKVEP KSC |
| 16 | Zalutumumab-VL+CL | AIQLTQSPSSLSASVGDRVTITCRASQDISSALVWYQQKPGKAPKLL IYDASSLESGVPSRFSGSESGTDFTLTISSLQPEDFATYYCQQFNSYP LTFGGGTKVEIKRTVAAPSVFIFPPSDEQLKSGTASVVCLLNNFYPR EAKVQWKVDNALQSGNSQESVTEQDSKDSTYSLSSTLTLSKADYE KHKVYACEVTHQGLSSPVTKSFNRGEC |
| 17 | Pertuzumab-VH+CH1 | EVQLVESGGGLVQPGGSLRLSCAASGFTFTDYTMDWVRQAPGKG LEWVADVNPNSGGSIYNQRFKGRFTLSVDRSKNTLYLQMNSLRAE DTAVYYCARNLGPSFYFDYWGQGTLVTVSSASTKGPSVFPLAPSSK STSGGTAALGCLVKDYFPEPVTVSWNSGALTSGVHTFPAVLQSSGL YSLSSVVTVPSSSLGTQTYICNVNHKPSNTKVDKKVEPKSC |
| 18 | Pertuzumab-VL+CL | DIQMTQSPSSLSASVGDRVTITCKASQDVSIGVAWYQQKPGKAPKL LIYSASYRYTGVPSRFSGSGSGTDFTLTISSLQPEDFATYYCQQYYIY PYTFGQGTKVEIKRTVAAPSVFIFPPSDEQLKSGTASVVCLLNNFYP REAKVQWKVDNALQSGNSQESVTEQDSKDSTYSLSSTLTLSKADY EKHKVYACEVTHQGLSSPVTKSFNRGEC |
| 19 | Wild-type CH3 domain (EU 341-447) | GQPREPQVYTLPPSREEMTKNQVSLTCLVKGFYPSDIAVEWESNGQ |
| | | PENNYKTTPPVLDSDGSFFLYSKLTVDKSRWQQGNVFSCSVMHEA LHNHYTQKSLSLSPGK |
| 20 | Wild-type CH3 domain Lacking residue K447 (EU 341-446) | GQPREPQVYTLPPSREEMTKNQVSLTCLVKGFYPSDIAVEWESNGQ PENNYKTTPPVLDSDGSFFLYSKLTVDKSRWQQGNVFSCSVMHEA LHNHYTQKSLSLSPG |
| 21 | Wild-type CH2 domain (EU 231-340) | APELLGGPSVFLFPPKPKDTLMISRTPEVTCVVVDVSHEDPEVKFN WYVDGVEVHNAKTKPREEQYNSTYRVVSVLTVLHQDWLNGKEY KCKVSNKALPAPIEKTISKAK |
| 22 | Linker | GGGSGGGS |
| 23 | Linker | GSTSGSGKPGSGEGSTKG |
| 24 | Linker | GGGSGGGSGGGS |
| 25 | Linker | GGGGSGGGGSGGGGS |
| 26 | Linker | GGGSGGGSGGGSGGGS |
| 27 | Linker | GGGGSGGGGSGGGGSGGGGS |
| 28 | Linker | GGGSGGGSGGGSGGGSGGGS |
| 29 | Linker | GGGGSGGGGSGGGGSGGGGSGGGGS |
| 30 | Linker | GGGSGGGSGGGSGGGSGGGSGGGS |
| 31 | Linker | GGGGSGGGGSGGGGSGGGGSGGGGSGGGGS |
| 32 | Linker | GGG |
| 33 | Linker | DGGGS |
| 34 | Linker | TGEKP |
| 35 | Linker | GGRR |
| 36 | Linker | EGKSSGSGSESKVD |
| 37 | Linker | KESGSVSSEQLAQFRSLD |
| 38 | Linker | GGRRGGGS |
| 39 | Linker | LRQRDGERP |
| 40 | Linker | LRQKDGGGSERP |

## Claims

1. A heterodimeric Fc scaffold comprising a first polypeptide and a second polypeptide,
wherein the first polypeptide comprises a first Fc region and the second polypeptide comprises a second Fc region,
wherein the first Fc region comprises a first CH3 domain and the second Fc region comprises a second CH3 domain,
wherein, relative to a wild-type CH3 domain, the first Fc region and the second Fc region comprise, in their respective CH3 domains, a knob mutation and a hole mutation of knobs-into-holes (KIH) mutations, respectively; and wherein:
(a) the Fc region comprising the hole mutation further comprises an L351Y mutation; or
(b) the Fc region comprising the hole mutation further comprises L351Y and D399R mutations, and the Fc region comprising the knob mutation further comprises a K409D mutation;
wherein the first polypeptide and the second polypeptide pair and heterodimerize via the first and second Fc regions to form the Fc scaffold;
wherein amino acid residues are numbered according to Kabat EU numbering.

2. The heterodimeric Fc scaffold of claim 1, wherein the first and second Fc regions comprise CH2 and CH3 domains, or comprise a hinge region, CH2 and CH3 domains.

3. The heterodimeric Fc scaffold of any one of claims 1-2, wherein the first and second Fc regions are of IgG class, e.g., IgG1, IgG2, IgG3 or IgG4 subclass; preferably, the first and second Fc regions are of human IgG1 subclass.

4. The heterodimeric Fc scaffold of any one of claims 1-3, wherein the knob mutation is T366W; and the hole mutation is Y407V or T366S-L368A-Y407V;
wherein, preferably,
the first Fc region comprises T366W and the second Fc region comprises T366S-L368A-Y407V-L351Y; or
the first Fc region comprises T366W-K409D and the second Fc region comprises T366S-L368A-Y407V-L351Y-D399R.

5. The heterodimeric Fc scaffold of any one of claims 1-4, wherein:
(a) relative to a wild-type CH3 domain, each of the first and second CH3 domains further comprises 0 to 3 amino acid residue changes; preferably, the wild-type CH3 domain is a CH3 domain of a native human IgG1 immunoglobulin, and more preferably comprises the amino acid sequence of SEQ ID NO: 19 or 20;
(b) the first and second Fc regions comprise a CH2 domain of a native human IgG1 immunoglobulin, or a CH2 domain comprising the amino acid sequence of SEQ ID NO: 21, or comprising 1 to 5 amino acid residue changes relative thereto;
(c) the Fc region comprising the knob mutation comprises the amino acid sequence of SEQ ID NO: 1 or 2, or an amino acid sequence having at least 95%, 96%, 97%, 98%, or 99% sequence identity thereto;
(d) the Fc region comprising the hole mutation comprises the amino acid sequence of SEQ ID NO: 7 or 8, or an amino acid sequence having at least 95%, 96%, 97%, 98%, or 99% sequence identity thereto; and/or
(e) the Fc scaffold does not form a non-naturally occurring disulfide bond between the first Fc region and the second Fc region.

6. The heterodimeric Fc scaffold of any one of claims 1-5, wherein the Fc scaffold has increased thermal stability compared to a corresponding control Fc scaffold comprising only the KIH mutations, and preferably, as determined by DSC, the CH3 domains of the Fc scaffold have a Tm of about 70°C or higher.

7. A CH3 heterodimer comprising a first immunoglobulin CH3 domain and a second immunoglobulin CH3 domain that are heterodimerized, wherein, relative to a wild-type CH3 domain, the first CH3 domain and the second CH3 domain comprise, respectively, a knob mutation and a hole mutation of knobs-into-holes (KIH) mutations; and wherein:
(a) the CH3 domain comprising the hole mutation further comprises an L351Y mutation; or
(b) the CH3 domain comprising the hole mutation further comprises L351Y and D399R mutations, and the CH3 domain comprising the knob mutation further comprises a K409D mutation;
wherein amino acid residues are numbered according to Kabat EU numbering;
preferably, the first and second CH3 domains are of IgG class, e.g., IgG1, IgG2, IgG3, or IgG4 subclass;
preferably, the wild-type CH3 domain is a CH3 domain of a native human IgG1 immunoglobulin; and
more preferably, relative to the wild-type CH3 domain, each of the first and second CH3 domains further comprises 0 to 3 amino acid residue changes.

8. A heteromultimeric protein comprising the heterodimeric Fc scaffold of any one of claims 1-6 or the CH3 heterodimer of claim 7.

9. A binding protein comprising the heterodimeric Fc scaffold of any one of claims 1-6 linked to at least one target-binding domain.

10. The binding protein of claim 9, wherein the at least one target-binding domain is each independently selected from: a ligand, a receptor-binding domain of a ligand, a ligand-binding domain of a receptor, and an antigen-binding domain of an antibody.

11. The binding protein of any one of claims 9-10, wherein the binding protein is monospecific or bispecific.

12. The binding protein of any one of claims 9-11, wherein the binding protein comprises at least one target-binding domain, each independently selected from the group consisting of an Fv, an scFv, a Fab, an scFab, a CrossFab, a VHH, and a ligand; preferably 1 to 6 said target-binding domains; more preferably 1, 2, or 3 said target-binding domains.

13. The binding protein of any one of claims 9-12, wherein the binding protein comprises only one said heterodimeric Fc scaffold.

14. The binding protein of any one of claims 9-13, wherein the binding protein is in an IgG format or an IgG-like format,
preferably wherein the binding protein has a format selected from: scFv-Fc/scFv-Fc, Fab-Fc/Fab-Fc, VHH-Fc/VHH-Fc, VHH-Fc/Fab-Fc, VHH-Fc/scFv-Fc, VHH-Fc/Fc, scFv-Fc/Fc, Fab-Fc/Fc, ligand-Fc/ligand-Fc, ligand-Fc/scFv-Fc, ligand-Fc/VHH-Fc, ligand-Fc/Fab-Fc, and ligand-Fc/Fc.

15. The binding protein of any one of claims 9-14, wherein the binding protein is bispecific and comprises:
(a) two different Fab domains respectively linked to the N-termini of the first and second Fc regions of said heterodimeric Fc scaffold, wherein the Fab domains comprise the same or different VL-CL light chains; or
(b) a VHH domain and a Fab domain respectively linked to the N-termini of the first and second Fc regions of said heterodimeric Fc scaffold.

16. The binding protein of any one of claims 9-15, having one or more of the following properties:
(a) thermal stability, wherein the CH3 domain has a Tm value of greater than or equal to 74 °C as determined by DSC;
(b) a purity of 90% or higher;
(c) binding affinity to an FcRn receptor, which is not less than 80% of the binding affinity of a native IgG1 monoclonal antibody to the FcRn receptor, preferably not less than 85%, 90%, or 95%;
(d) binding affinity to an FcγR1 receptor, which is not less than 50% of the binding affinity of a native IgG1 monoclonal antibody to the FcγR1 receptor; and/or
(e) storage stability, wherein after storage at 42 °C for 2 weeks, a change in purity is less than 2% as determined by SEC-HPLC.

17. A polynucleotide encoding the heterodimeric Fc scaffold of any one of claims 1-6, the CH3 heterodimer of claim 7, the heteromultimeric protein of claim 8, or the binding protein of any one of claims 9-16.

18. A vector or host cell comprising the polynucleotide of claim 17.

19. A composition comprising the heteromultimeric protein of claim 8 or the binding protein of any one of claims 9-16.

20. A conjugate comprising the heteromultimeric protein of claim 8 or the binding protein of any one of claims 9-16 conjugated to a therapeutic or diagnostic agent.

21. Use of the composition of claim 19 or the conjugate of claim 20 as a medicament, or in the manufacture of a medicament, preferably the medicament is for use in the treatment of cancer.
